# EUROPÄISCHE PATENTSCHRIFT

(11) **EP 3 063 525 B1**
(45) Veröffentlichungstag und Bekanntmachung des Hinweises auf die Patenterteilung: **02.06.2021**
(21) Anmeldenummer: 14805486.9
(22) Anmeldetag: 27.10.2014
(51) Int. Cl.: G01N 1/28, G01N 33/53, G02B 21/34, G01N 1/31

(54) **VERBESSERTE VORRICHTUNG UND VERFAHREN FÜR REAKTIONEN ZWISCHEN EINER FESTEN UND EINER FLÜSSIGEN PHASE**
IMPROVED DEVICE AND METHOD FOR REACTIONS BETWEEN A SOLID AND A LIQUID PHASE
DISPOSITIF AMÉLIORÉ ET PROCÉDÉ DE CONDUITE DE RÉACTIONS ENTRE UNE PHASE SOLIDE ET UNE PHASE LIQUIDE

(30) Priorität: 28.10.2013 DE 102013017802; 06.02.2014 DE 102014001481
(43) Veröffentlichungstag der Anmeldung: 07.09.2016
(73) Patentinhaber: Euroimmun Medizinische Labordiagnostika AG, 23560 Lübeck (DE)
(72) Erfinder: STÖCKER, Winfried, 23627 Groß Grönau (DE)
(86) Internationale Anmeldenummer: PCT/EP2014/002889
(87) Internationale Veröffentlichungsnummer: WO 2015/062715

(56) Entgegenhaltungen:
- EP-A1- 0 117 262
- EP-A1- 2 191 893
- EP-A2- 0 270 363
- EP-A2- 2 053 378
- WO-A1-01/04634
- US-A- 4 274 359
- US-A- 5 068 091
- US-A- 5 338 358
- US-A- 5 451 500
- US-A1- 2006 239 858
- US-A1- 2012 149 050

## Beschreibung

Die vorliegende Erfindung betrifft eine Vorrichtung zum Kontaktieren eines immobilisierten Reaktionspartners mit wenigstens einer Flüssigkeit, umfassend eine Wanne mit einem Boden, aus dem wenigstens eine Erhebung mit einer oberen Seite in den Innenraum der Wanne ragt, wobei die wenigstens eine Erhebung einen Kanal aufweist, der vom Boden der Wanne zur oberen Seite der Erhebung, bevorzugt senkrecht zum Boden der Wanne verläuft und auf der oberen Seite der Erhebung in wenigstens eine Austrittsöffnung mündet, wobei der Kanal über ein Zufuhrelement mittels einer Pumpvorrichtung aus wenigstens einem Flüssigkeitsreservoir gespeist wird, das die wenigstens eine Flüssigkeit enthält, eine Halterungsvorrichtung, die eingerichtet ist, um wenigstens einen Objektträger, bevorzugt eine Vielzahl von Objektträgern, aufweisend wenigstens eine Adhäsionsfläche mit dem immobilisierten Reaktionspartner, lösbar derart zu halten, dass die Adhäsionsfläche dem Innenraum der Wanne zugewandt ist, und dass die obere Seite der Erhebung und die Adhäsionsfläche derart relativ zueinander positioniert sind, dass aus der Austrittsöffnung austretende Flüssigkeit mit dem immobilisierten Reagenz in Kontakt steht, wobei die Vorrichtung optional einen in die Halterungsvorrichtung eingebrachten Objektträger umfasst, wobei die obere Seite der Erhebung und die Adhäsionsfläche parallel zueinander angeordnet sind, und wobei der Objektträger wenigstens zwei voneinander abgegrenzte Adhäsionsflächen aufweist oder die Halterungsvorrichtung als separates Teil ausgestattet ist, das für die Aufnahme und das Halten von wenigstens zwei Objektträgern ausgestattet ist, charakterisiert dadurch, dass die Vorrichtung in der einen Wanne zwei oder mehr Erhebungen und entsprechend zwei oder mehr korrespondierende Adhäsionsflächen aufweist, und dass diese derart gestaltet sind und sich in einem ausreichendem Abstand voneinander befinden, dass die durch die Erhebungen eingeleitete Flüssigkeit nicht mit Adhäsionsflächen in Berührung kommen, die benachbarten Erhebungen gegenüber liegen. Die Erfindung betrifft ferner ein Verfahren zum Kontaktieren eines immobilisierten Reaktionspartners mit wenigstens einer Flüssigkeit, umfassend die Schritte a) Immobilisieren des Reaktionspartners auf der Adhäsionsfläche wenigstens eines Objektträgers, der zum Einbringen in die Halterungsvorrichtung der erfindungsgemäßen Vorrichtung geeignet ist, b) sofern die vorherigen Schritte außerhalb der erfindungsgemäßen Vorrichtung durchgeführt wurden: Einbringen des Objektträgers in die Halterungsvorrichtung der erfindungsgemäßen Vorrichtung, bevorzugt über eine Transportvorrichtung in der erfindungsgemäßen Vorrichtung, c) Einleiten der wenigstens einen Flüssigkeit über das Zufuhrelement in Richtung der Austrittsöffnung, bis die wenigstens eine Flüssigkeit mit dem immobilisierten Reaktionspartner in Kontakt steht.

Zahlreiche analytische Verfahren auf den Gebieten der Chemie, Biologie und Medizin, besonders im Bereich der labormedizinischen Diagnostik, beruhen darauf, dass ein einzelner Reaktand, beispielsweise ein Autoantikörper, in einem komplexen Probengemisch über seine spezifische Wechselwirkung mit einem Reaktionspartner, beispielsweise einem Antigen, nachgewiesen wird. Diese spezifische Wechselwirkung lässt sich am effizientesten dann nachweisen, wenn andere Stoffe neben dem Reaktanden und seinem Reaktionspartner in möglichst geringen Konzentrationen anwesend sind. Aus diesem Grund immobilisiert man den Reaktionspartner häufig über eine kovalente oder reversible chemische Bindung, inkubiert ihn anschließend mit dem Probengemisch, das den einzelnen Reaktanden enthalten könnte, und entfernt schließlich das um den Reaktanden abgereicherte Probengemisch, so dass der Komplex aus dem nachzuweisenden Reaktanden und dem Reaktionspartner übrig bleibt. Dieser Komplex kann mit üblichen Nachweisverfahren wie Fluoreszenz, Spektroskopie oder Farbreaktionen detektiert werden.

Ein Beispiel für ein derartiges analytisches Nachweisverfahren ist der klassische ELISA (Enzyme-Linked Immunosorbent Assay), der ausgeführt werden kann, indem zunächst ein beliebiger, von einem Antikörper erkannter Reaktionspartner immobilisiert wird, beispielsweise ein Peptid oder ein Protein. Anschließend wird ein Probengemisch, das den nachzuweisenden Antikörper enthalten könnte, mit dem immobilisierten Reaktionspartner in wässriger Lösung unter Bedingungen kontaktiert, die einer Komplexbildung förderlich sind. Nach Entfernung des Probengemisches wird der Komplex zwischen dem immobilisierten Antigen und dem Antikörper über einen zweiten, enzymmarkierten Antikörper nachgewiesen, der eine Farbreaktion katalysieren kann.

Für die Empfindlichkeit und Spezifität des Nachweises ist die Reinheit des Komplexes aus immobilisiertem Reaktionspartner und Reaktand entscheidend. Zusätzliche, kontaminierende Stoffe aus dem Probengemisch können den physikalischen Nachweis des Komplexes stören, indem sie z. B. bei Fluoreszenzmessungen in ähnlichen Wellenlängenbereichen emittieren wie der Komplex. Auch ein chemischer Abbau des Komplexes ist möglich. Beispielsweise enthält menschliches Serum reaktive Komponenten wie Proteasen, die Proteinbestandteile des Komplexes abbauen können. Es gilt daher, den Komplex möglichst effizient durch Waschschritte von kontaminierenden Stoffen zu befreien.

Während andere Schritte derartiger analytischer Verfahren, beispielsweise das Immobilisieren des Reaktionspartners und das Kontaktieren mit Nachweisreagenzien, mittlerweile automatisiert in Hochdurchsatzverfahren durchgeführt werden können, werden die Waschschritte, insbesondere bei komplexen Molekülen oder empfindlichen Geweben als immobilisierten Reaktionspartner, heutzutage noch weitgehend manuell durchgeführt. Zu diesem Zweck werden Objektträger üblicherweise von Hand dem Analyseinstrument entnommen und zusammen mit anderen Objektträgern aus parallelen Ansätzen in eine Wanne mit Waschlösung eingebracht.

Schon ein einzelner manuell durchgeführter Schritt verringert aber ganz erheblich die Effizienz der analytischen Hochdurchsatzverfahren. Da die Anwesenheit einer geschulten Fachkraft erforderlich ist, müssen die Arbeitsabläufe entsprechend aufwändig geplant werden. Es ist beispielsweise nicht möglich, das Verfahren unbeaufsichtigt über Nacht laufen zu lassen und lediglich das fertige Ergebnis zu einem beliebigen Zeitpunkt am nächsten Tag zur Kenntnis zu nehmen.

Die herkömmliche manuelle Fahrweise bei Waschschritten hat weitere Nachteile. Werden mehrere Objektträger in einer Wanne zusammen gewaschen, so besteht die Gefahr einer Kontamination, wenn nachzuweisende Reaktanden durch Diffusion auch mit immobilisierten Reaktionspartner in Kontakt geraten, die zuvor mit einer Probe kontaktiert wurden, die keinen Reaktanden enthielt. Dies kann zu einem hohen Fluoreszenzhintergrund, der die Auswertung erschwert, oder im schlimmsten Fall zu Fehlbefunden führen.

Das Volumen der Waschlösung muss so groß bemessen sein, dass sie den gesamten Objektträger bedeckt, was den Ressourcenbedarf des Verfahrens bei manuell durchgeführten Waschschritten erhöht.

Die einzelnen Objektträger müssen für eine Reihe von Nachweisverfahren zudem getrocknet werden, was wiederum überwiegend manuell geschieht. Die manuelle Fahrweise bedingt dabei die Möglichkeit, dass Trocknungsschritte zu spät, schlecht reproduzierbar oder unvollständig ausgeführt werden. Als Folge kann die Qualität des Reaktionspartners oder des Nachweisverfahrens leiden, wenn etwa bei verspätetem Trocknen verdunstende Pufferlösungen Salzkristalle ausbilden oder wässrige biologische Proben durch mikrobielle Besiedlung und Kontamination verderben.

Im Stand der Technik, beispielsweise in der US2006239858, sind Vorrichtungen beschrieben, mit denen ein Objektträger kopfüber, d. h. mit der Seite nach unten, auf der die zu untersuchende Probe immobilisiert ist, in eine Wanne mit Flüssigkeit eingetaucht wird. Solche Vorrichtungen erlauben nur die separate Bearbeitung einzelner Proben, ermöglichen aber keine Effizienzsteigerung bei paralleler Abarbeitung einer Vielzahl von Proben. Nicht zuletzt bleibt das Problem ungelöst, dass das Volumen der Lösung, in die der Objektträger einzutauchen ist, so groß sein muß, dass wenigstens eine Seite des Objektträgers vollständig bedeckt ist.

Das Dokument US 2012/149050 A1 offenbart eine Vorrichtung zum Kontaktieren eines immobilisierten Reaktionspartners mit einer Flüssigkeit, umfassend eine Wanne, aus dem eine Erhebung mit einer oberen Seite in den Innenraum der Wanne ragt, wobei die eine Erhebung einen Kanal aufweist, der vom Boden der Wanne zur oberen Seite der Erhebung verläuft und nach der oberen Seite der Erhebung in wenigstens einer Austrittsöffnung mündet. Der Kanal wird über ein Zufuhrelement mittels einer Pumpvorrichtung aus einem Reservoir gespeist, welches die Flüssigkeit enthält, wobei eine Halterungsvorrichtung eingerichtet ist, um einen Objektträger aufweisend eine Adhäsionsfläche mit einem immobilisierten Reaktionspartner lösbar verbunden zu halten, dass die Adhäsionsfläche dem Inneren der Wanne zugewandt ist und dass die obere Seite der Erhebung und die Adhäsionsfläche so zueinander positioniert sind, dass aus der Austrittsöffnung austretende Flüssigkeit mit dem immobilisierten Reagenz in Kontakt steht.

Das Dokument US 5,068,091 A offenbart eine Vorrichtung zum Färben von Geweben von lebenden Körpern für die Beobachtung von Immunantworten mit einem Färbeblock, auf dem eine Vielzahl von benachbarten Basisbereichen gebildet ist, die je eine flache obere Fläche aufweisen. Auf einer Seite des Basisbereiches ist eine Rippe und auf der anderen Seite des Basisbereichs ist eine Trageoberfläche gebildet, die leicht höher ist als die Rippe.

Das Dokument EP 2 053 378 A2 offenbart eine Vorrichtung zum Vorhalten einer Probe bzw. einer Platte, auch Objektträger genannt, wobei die Vorrichtung ein Reagenzienreservoir und eine Reaktionskammer aufweist. Eine Platte kann auf der Vorhaltevorrichtung positioniert werden, so dass eine Gewebeprobe nach unten gerichtet ist zwischen der Platte und einer Auflage der Halterungsvorrichtung. Ein Reagenz aus dem Reservoir wird in einen flüssigen Zustand gebracht und fließt vom Reservoir in die Reaktionskammer.

Das Dokument US 4,274,359 offenbart eine Halterungsvorrichtung umfassend einen Rahmen, der für die Aufnahme wenigstens eines Objektträgers ausgestattet ist, wobei sich eine Fläche des Objektträgers nach der Aufnahme auf der Seite des Objektträgers befindet, mit der er auf dem Rahmen aufliegt. Der Objektträger kann mit seiner drogentragenden Fläche sowohl auf der Oberseite oder auf der Unterseite auf dem Rahmen aufliegen.

Das Dokument US 5,338,358 A offenbart eine Vorrichtung zum Färben von einem lebenden Körper entnommen Gewebeproben zur Beobachtung der Immunreaktion der Gewebe weist einen Färbedruck mit einer Vielzahl von parallelen angeordneten Plateaus auf. Ein Objektträger ist über jedem Plateau so angeordnet, dass ein keilförmiger Spalt kapillarer Abmessungen zwischen dem Plateau und der Unterseite des Objektträgers, an der eine Gewebeprobe befestigt ist, gebildet ist. An einem Ende der Oberseite jedes Plateaus ist eine Flüssigkeitsdruckfläche und eine Flüssigkeitsablauföffnung ausgebildet.

Vor diesem Hintergrund besteht die der vorliegenden Erfindung zu Grunde liegende Aufgabe darin, eine Vorrichtung bereitzustellen, die es gestattet, einen immobilisierten Reaktionspartner in mehreren getrennten Ansätzen parallel jeweils nacheinander mit verschiedenen Flüssigkeiten, insbesondere mit einer Waschflüssigkeit, zu kontaktieren und anschließend zu trocknen, ohne dass es dabei zu einer Vermischung von Reagenzien oder Flüssigkeiten aus getrennten Ansätzen kommt.

Eine weitere, der vorliegenden Erfindung zu Grunde liegende Aufgabe besteht darin, eine Vorrichtung zur Durchführung analytischer Nachweisverfahren bereitzustellen, wobei der Platzbedarf, der Verbrauch an Flüssigkeit und/oder die Zeitdauer gegenüber den im Stand der Technik beschriebenen Verfahren verringert sind, wohingegen die Reproduzierbarkeit erhöht ist.

Eine weitere, der vorliegenden Erfindung zu Grunde liegende Aufgabe besteht darin, eine Vorrichtung zur Durchführung analytischer Nachweisverfahren bereitzustellen, wobei die Störanfälligkeit des Nachweises, beispielsweise durch in der Probe enthaltene Feststoffe oder durch Artefakte, verringert ist, besonders für den Fall, dass der Nachweis mittels Immunfluoreszenz durchgeführt wird.

Diese und weitere Aufgaben werden durch den Gegenstand der vorliegenden Anmeldung und insbesondere auch durch den Gegenstand der beigefügten unabhängigen Ansprüche gelöst, wobei sich Ausführungsformen aus den Unteransprüchen ergeben.

Die der Erfindung zu Grunde liegende Aufgabe wird in einem ersten Aspekt gelöst durch eine Vorrichtung zum Kontaktieren wenigstens eines immobilisierten Reaktionspartners mit wenigstens einer Flüssigkeit, umfassend eine Wanne mit einem Boden, aus dem wenigstens eine Erhebung mit einer oberen Seite in den Innenraum der Wanne ragt, wobei die wenigstens eine Erhebung einen Kanal aufweist, der vom Boden der Wanne zur oberen Seite der Erhebung, bevorzugt senkrecht zum Boden der Wanne verläuft und auf der oberen Seite der Erhebung in wenigstens eine Austrittsöffnung mündet, wobei der Kanal über ein Zufuhrelement mittels einer Pumpvorrichtung aus wenigstens einem Flüssigkeitsreservoir gespeist wird, das die wenigstens eine Flüssigkeit enthält, eine Halterungsvorrichtung, die eingerichtet ist, um wenigstens einen Objektträger aufweisend wenigstens eine Adhäsionsfläche mit dem wenigstens einen immobilisierten Reagenz lösbar derart zu halten, dass die Adhäsionsfläche dem Innenraum der Wanne zugewandt ist, wobei die obere Seite der Erhebung und die Adhäsionsfläche derart zueinander positioniert sind, und dass aus der Austrittsöffnung austretende Flüssigkeit mit dem immobilisierten Reagenz in Kontakt steht,
wobei die Vorrichtung wenigstens einen in die Halterungsvorrichtung eingebrachten Objektträger umfasst, wobei die obere Seite der Erhebung und die Adhäsionsfläche parallel zueinander angeordnet sind, und wobei der Objektträger wenigstens zwei voneinander abgegrenzte Adhäsionsflächen aufweist oder die Halterungsvorrichtung als separates Teil ausgestattet ist, das für die Aufnahme und das Halten von wenigstens zwei Objektträgern ausgestattet ist, charakterisiert dadurch, dass die Vorrichtung in der einen Wanne zwei oder mehr Erhebungen und entsprechend zwei oder mehr korrespondierende Adhäsionsflächen aufweist, und dass diese derart gestaltet sind und sich in einem ausreichendem Abstand voneinander befinden, dass die durch die Erhebungen eingeleitete Flüssigkeit nicht mit Adhäsionsflächen in Berührung kommen, die benachbarten Erhebungen gegenüber liegen.

In einer ersten Ausführungsform des ersten Aspektes wird das Problem gelöst durch eine Vorrichtung, wobei der Kanal zusätzlich aus einem weiteren Flüssigkeitsreservoir gespeist wird, das eine zweite Flüssigkeit enthält.

In einer zweiten Ausführungsform, die auch eine Ausführungsform der ersten Ausführungsform des ersten Aspektes darstellt, wird das Problem gelöst durch eine Vorrichtung, wobei der Kanal in mehr als eine Austrittöffnung auf der oberen Seite der Erhebung mündet.

In einer dritten Ausführungsform, die auch eine Ausführungsform der ersten bis zweiten Ausführungsform des ersten Aspekts darstellt, wird das Problem gelöst durch eine Vorrichtung, wobei die Adhäsionsfläche einen Mittelpunkt aufweist und die obere Seite der Erhebung einen Mittelpunkt aufweist, und wobei sich Mittelpunkt der Adhäsionsfläche und Mittelpunkt der oberen Seite der Erhebung gegenüber liegen.

In einer vierten Ausführungsform, die auch eine Ausführungsform der ersten bis dritten Ausführungsform des ersten Aspekts darstellt, wird das Problem gelöst durch eine Vorrichtung, wobei die Wanne wenigstens eine Ablauföffnung aufweist.

In einer fünften Ausführungsform, die auch eine Ausführungsform der ersten bis vierten Ausführungsform des ersten Aspekts darstellt, wird das Problem gelöst durch eine Vorrichtung, wobei die Halterungsvorrichtung eine Verschiebung des Objektträgers, bevorzugt entlang der Längsachse der Wanne, gestattet, bevorzugt über wenigstens eine an den oberen Rändern der Wanne angebrachte Rinne oder Schiene.

In einer sechsten Ausführungsform, die auch eine Ausführungsform der ersten bis fünften Ausführungsform des ersten Aspekts darstellt, wird das Problem gelöst durch eine Vorrichtung, wobei die Wanne weiterhin mit wenigstens einer Luftdüse zum Trocknen der Adhäsionsfläche ausgestattet ist, und wobei bevorzugt der Luftstrahl aus der wenigstens einen Luftdüse mit der durch den Boden der Wanne festgelegten Ebene einen Winkel (W) von weniger als 90 Grad, bevorzugt 20 bis 70 Grad bildet.

In einer siebten Ausführungsform, die auch eine Ausführungsform der ersten bis sechsten Ausführungsform des ersten Aspekts darstellt, wird das Problem gelöst durch eine Vorrichtung, wobei die Pumpvorrichtung gestattet, dass Flüssigkeit, die mit dem immobilisierten Reaktionspartner in Kontakt steht, aus Richtung der Austrittsöffnung abgesaugt wird.

In einer achten Ausführungsform, die auch eine Ausführungsform der ersten bis siebten Ausführungsform des ersten Aspekts darstellt, wird das Problem gelöst durch eine Vorrichtung, weiter umfassend eine Transportvorrichtung, die das Einbringen des Objektträgers in die Vorrichtung gestattet.

In einer neunten Ausführungsform, die auch eine Ausführungsform der ersten bis achten Ausführungsform des ersten Aspekts darstellt, wird das Problem gelöst durch eine Vorrichtung, wobei die Austrittsöffnung auf der oberen Seite der Erhebung, die obere Seite der Erhebung und die Adhäsionsfläche derart ausgestaltet sind, dass die wenigstens eine Flüssigkeit tangential am immobilisierten Reaktionspartner entlang strömt, wenn sie in die Vorrichtung eingeleitet wird.

In einer zehnten Ausführungsform, die auch eine Ausführungsform der ersten bis neunten Ausführungsform ist, umfasst die Halterungsvorrichtung einen Rahmen, der für die Aufnahme und das Halten des wenigstens Objektträgers, bevorzugt einer Vielzahl von Objektträgern, ausgestattet ist,
wobei sich die Adhäsionsfläche nach der Aufnahme auf der Seite des Objektträgers befindet, mit der er auf dem Rahmen aufliegt.

Vorgeschlagen wird ferner eine bevorzugte Halterungsvorrichtung umfassend einen Rahmen, der für die Aufnahme wenigstens eines Objektträgers, bevorzugt einer Vielzahl von Objektträgern, ausgestattet ist,
wobei der Objektträger wenigstens eine Adhäsionsfläche mit einem immobilisierten Reaktionspartner aufweist,
wobei sich die Adhäsionsfläche des Objektträgers nach der Aufnahme auf der Seite des Objektträgers befindet, mit der er auf dem Rahmen aufliegt,
und wobei die Adhäsionsfläche nach der Aufnahme derart positioniert ist, dass sie einem Kontakt mit aus der Austrittsöffnung austretender Flüssigkeit zugänglich ist.

Erfindungsgemäß umfasst die Halterungsvorrichtung einen Objektträger.

In einer bevorzugten Ausführungsform weist der Rahmen wenigstens eine Seitenwand auf, die bevorzugt mit einem Griff ausgestattet ist, wobei die Seitenwand des Rahmens noch bevorzugter ein Profil aufweist, das zum Positionieren der Halterungsvorrichtung und damit der Adhäsionsfläche in der Vorrichtung geeignet ist.

Die der Erfindung zu Grunde liegende Aufgabe wird in einem zweiten Aspekt gelöst durch ein Verfahren zum Kontaktieren wenigstens eines immobilisierten Reaktionspartners mit wenigstens einer Flüssigkeit, umfassend die Schritte a) Bereitstellen der erfindungsgemäßen Vorrichtung umfassend den wenigstens einen Objektträger mit immobilisiertem Reaktionspartner, und b) Einleiten der wenigstens einen Flüssigkeit über das Zufuhrelement in Richtung der Austrittsöffnung, bis die wenigstens eine Flüssigkeit mit dem immobilisierten Reaktionspartner in Kontakt steht.

In einer ersten Ausführungsform des zweiten Aspekts wird das Problem gelöst durch ein Verfahren umfassend den Schritt c) Einleiten einer zweiten Flüssigkeit und optional einer weiteren Flüssigkeit über das Zufuhrelement in Richtung der Austrittsöffnung, bis die zweite Flüssigkeit mit dem immobilisierten Reaktionspartner in Kontakt steht.

In einer zweiten Ausführungsform, die auch eine Ausführungsform der ersten Ausführungsform des zweiten Aspekts darstellt, wird das Problem gelöst durch ein Verfahren weiter umfassend den Schritt d) Einleiten einer Waschlösung über das Zufuhrelement in Richtung der Austrittsöffnung, bis die Waschlösung mit dem immobilisierten Reaktionspartner in Kontakt steht, wobei Schritt d) vor und/oder nach jedem der Schritte a), b), und c) ausgeführt werden kann.

In einer dritten Ausführungsform, die eine Ausführungsform der zweiten Ausführungsform des zweiten Aspekts darstellt, wird das Problem gelöst durch ein Verfahren, wobei das Einleiten der Waschlösung durchgeführt wird, bis die erfindungsgemäße Vorrichtung vollständig von Verunreinigungen befreit ist.

In einer dritten Ausführungsform, die auch eine Ausführungsform der ersten bis zweiten Ausführungsform des zweiten Aspekts darstellt, wird das Problem gelöst durch ein Verfahren, wobei das Einleiten der wenigstens einen Flüssigkeit, der zweiten Flüssigkeit und optional weiteren Flüssigkeit und/oder Waschlösung, bevorzugt das Einleiten sowohl der wenigstens einen und zweiten Flüssigkeit als auch der Waschlösung, gefolgt wird von einem zusätzlichen Schritt e) Absaugen der wenigstens einen oder zweiten Flüssigkeit und optional weiteren Flüssigkeit oder der Waschlösung von dem immobilisierten Reaktionspartner aus Richtung der Adhäsionsfläche über den Kanal.

In einer vierten Ausführungsform, die auch eine Ausführungsform der ersten bis dritten Ausführungsform des zweiten Aspekts darstellt, umfasst Schritt a) das Aufnehmen des wenigstens einen Objektträgers in die erfindungsgemäße Halterungsvorrichtung umfassend einen Rahmen und das nachfolgende Einbringen der Halterungsvorrichtung in die erfindungsgemäße Vorrichtung.

Die vorliegende Erfindung betrifft ein Verfahren zum Kontaktieren wenigstens eines immobilisierten Reaktionspartners mit wenigstens einer Flüssigkeit, bevorzugt für immunologische, histo- und cytochemische, molekularbiologische, enzymologische, pharmakologische und klinisch-chemische Nachweisverfahren, und erfordert zunächst eine Wanne. In einer bevorzugten Ausführungsform handelt es sich bei der Wanne um ein Gefäß beliebiger Bauart mit einem Boden, der bevorzugt von aufrecht, bevorzugt senkrecht, stehenden Wänden umgeben ist, die gegenüber der Umgebung einen Innenraum der Wanne abschließen, das geeignet ist, einerseits über die Halterungsvorrichtung den Objektträger, andererseits die in den eigenen Innenraum ragenden Erhebungen zu beherbergen. Beispielsweise ist die Wanne eine Wanne mit rechteckiger Grundfläche, eine Schale oder eine Dose. Bevorzugt ist die Wanne wasserdicht, so dass im Innenraum der Wanne verspritzte oder von den Erhebungen heruntertropfende Flüssigkeit nicht unkontrolliert entweichen kann. Typischerweise wird die Wanne aus einem chemisch reaktionsträgen, leicht zu reinigendem und zu verarbeitendem Kunststoff hergestellt, beispielsweise aus Plexiglas. Der Boden kann relativ zum Schwerefeld der Erde derart geneigt sein, dass auftreffende Flüssigkeit der Neigung entsprechend abfließt, bevorzugt in Richtung einer optional vorhandenen Auslassöffnung. Die Wanne kann offen oder mit einem Deckel, einer Klappe o. ä. verschließbar sein. Für lichtempfindliche immobilisierte Reaktionspartner, Reaktanden oder Flüssigkeiten kann die Wanne intransparent sein.

Der erfindungsgemäß zu verwendende Objektträger und die Wanne sind funktionell aneinander angepasst. In einer bevorzugten Ausführungsform wird unter dem Begriff "Objektträger", wie hierin verwendet, ein an die Wanne angepasstes Strukturelement verstanden, das dazu dient, den immobilisierten Reaktionspartner in einer für eingeleitete Flüssigkeit zugänglichen Form zu präsentieren. Geeignete Objektträger sind beispielsweise in der DE 20 2011 005 278 beschrieben. Typischerweise umfasst der Objektträger erfindungsgemäß einen Grundkörper aus Glas, prinzipiell sind aber auch andere Träger mit abweichenden Formen und aus anderen Materialien, beispielsweise Kunststoffen, verwendbar. Der Objektträger weist wenigstens zwei, bevorzugt wenigstens drei, vier, fünf, sechs, acht, 10, 12, 16, 20, 30, 40 oder 50 voneinander abgegrenzte Adhäsionsflächen auf. In einer bevorzugten Ausführungsform wird unter dem Begriff "Adhäsionsfläche", wie hierin verwendet, ein Feld auf dem Objektträger verstanden, das zur Immobilisierung des Reaktionspartners geeignet ist, direkt oder über einen zusätzlichen Träger. Die Adhäsionsfläche kann zu diesem Zweck reaktive oder aktivierbare chemische Funktionen aufweisen, die für die Ausbildung kovalenter Bindungen zu einem zu immobilisierenden Reaktionspartner geeignet sind. Alternativ kann die Adhäsionsfläche so beschaffen sein, dass der zu immobilisierende Reaktionspartner über hydrophobe Wechselwirkungen immobilisiert wird. Schließlich kann die Immobilisierung dadurch erfolgen, dass ein weiterer Träger, beispielsweise ein Biochip, auf der Adhäsionsfläche befestigt wird, beispielsweise durch Ankleben. Die Herstellung von derartigen Biochips ist im Stand der Technik beschrieben, beispielsweise in der EP 0 117 262, ebenso Vorrichtungen und Verfahren zur weitgehend automatisierten Herstellung von geeigneten Objektträgern, beispielsweise in der WO2005073693. Geometrisch kann die Adhäsionsfläche in der durch die Grundfläche des Objektträgers festgelegten Ebene, ihr gegenüber aber auch erhöht oder erniedrigt liegen. Die Adhäsionsfläche liegt bevorzugt parallel zur Grundfläche des Objektträgers.

Der Objektträger und die Wanne können erfindungsgemäß über eine Haltevorrichtung miteinander in Kontakt gehalten werden, die bewirkt, dass sie in einer definierten, festgelegten Position zueinander stehen, insbesondere mit festgelegtem Abstand. Zu diesem Zweck können Objektträger und Wanne einander zugeordnete Vorsprünge und Vertiefungen, Rastelemente und/oder Anschläge umfassen. Die Tatsache, dass der Objektträger kopfüber, d. h. mit der Adhäsionsfläche dem Boden der Wanne zugewandt liegt, bedingt, dass Feststoffe in einer Lösung, die mit der Adhäsionsfläche in Kontakt steht, durch die Schwerkraft sinken und, besonders bei gerichteter Strömung durch eingeleitete Flüssigkeit, nicht den Komplex aus immobilisiertem Reaktionspartner und Reaktand kontaminieren. Weiterhin können Objektträger und Wanne über geeignete Mittel lösbar miteinander verbunden sein, beispielsweise Klemmen, Verschlussknöpfe oder dergleichen. In einer bevorzugten Ausführungsform sind Objektträger und Wanne über die Haltevorrichtung nicht irreversibel aneinander befestigt, sondern voneinander lösbar zusammengehalten.

Ein weiteres essentielles Element der Erfindung ist die wenigstens eine vom Boden der Wanne in dessen Innenraum ragende Erhebung mit der Austrittsöffnung, über die die wenigstens eine Flüssigkeit aus dem Flüssigkeitsreservoir über das Zufuhrelement und den Kanal auf die obere Seite der Erhebung gelangen kann. Die obere Seite dieser Erhebung befindet sich nach dem Einbringen des Objektträgers in der Nähe der Adhäsionsfläche, bevorzugt in einem geringen Abstand, der eine gleichzeitige Benetzung der oberen Seite der Erhebung und der Adhäsionsfläche durch aus der Austrittsöffnung austretende Flüssigkeit gestattet, besonders bevorzugt derart, dass der Zwischenraum, wenn die Adhäsionsfläche mit einer Flüssigkeit in Kontakt steht, zwischen oberer Seite der Erhebung und Adhäsionsfläche vollständig mit Flüssigkeit gefüllt sein kann. Beispielsweise beträgt der Abstand zwischen oberer Seite der Erhebung und Adhäsionsfläche 0,05 bis 3,00 mm. Bevorzugt sind die obere Seite der Erhebung und die Adhäsionsfläche hydrophil und vollständig durch wässrige Flüssigkeit benetzbar. Weiterhin ist es erfindungsgemäß vorgesehen, dass die obere Seite der Erhebung und die Adhäsionsfläche parallel zueinander angeordnet und bevorzugt planar sind. In einer bevorzugten Ausführungsform weist die erfindungsgemäße Vorrichtung eine Vielzahl derartiger Erhebungen auf und kann eine entsprechende Zahl von Adhäsionsflächen gleichzeitig prozessieren, beispielsweise wenigstens oder genau 2, 4, 8, 16, 24, 30, 40, 50, 100, 200, 250, 300, 400, 500, 600, 700, 750, 800, 900 oder 1000 Erhebungen. Erfindungsgemäß weist die erfindungsgemäße Vorrichtung zwei oder mehr Erhebungen und entsprechend zwei oder mehr korrespondierende Adhäsionsflächen auf, und diese sind derart gestaltet und befinden sich in einem ausreichendem Abstand voneinander, dass die durch die Erhebungen eingeleitete Flüssigkeit nicht mit Adhäsionsflächen in Berührung kommen, die benachbarten Erhebungen gegenüber liegen.

Die erfindungsgemäße Vorrichtung ist so beschaffen, dass aus der Austrittsöffnung auf der oberen Seite der Erhebung austretende Flüssigkeit mit der Adhäsionsfläche in Kontakt tritt. In einer bevorzugten Ausführungsform wird unter dem Begriff "in Kontakt treten" verstanden, dass austretende Flüssigkeit mit dem immobilisierten Reaktionspartner derart in Kontakt steht, dass chemische Reaktionen zwischen einem in der Flüssigkeit enthaltenen Reaktanden und dem immobilisierten Reaktionspartner ablaufen können. In einer besonders bevorzugten Ausführungsform steht die Flüssigkeit dabei gleichzeitig mit der oberen Seite der Erhebung und mit der Adhäsionsfläche in Kontakt. In einer weiteren bevorzugten Ausführungsform steht die Flüssigkeit zum Zeitpunkt der Reaktion eines darin enthaltenen Reaktanden mit dem immobilisierten Reaktionspartner, nicht aber mit der oberen Seite der Erhebung in Kontakt. Das ist beispielsweise der Fall, wenn die Austrittsöffnung, der Abstand zwischen oberer Seite der Erhebung und Adhäsionsfläche und der Druck, mit dem die Flüssigkeit aus der Austrittsöffnung austritt, so bemessen sind, dass die Flüssigkeit in einem Strahl austritt und die Adhäsionsfläche besprüht wird.

Die Erhebung mit der Austrittsöffnung kann auf dem Boden der Wanne statisch oder mobil befestigt sein. Im Fall einer mobilen Erhebung kann die Erhebung mehrere Adhäsionsflächen, oder eine längliche Adhäsionsfläche sequentiell oder nacheinander abfahren und jeweils mit der wenigstens einen Flüssigkeit kontaktieren.

Die obere Seite der Erhebung kann eine, aber auch mehr als eine Austrittsöffnung umfassen. Ist Letzteres der Fall, so ist bevorzugt, dass die Austrittsöffnungen gleichmäßig auf der oberen Seite der Erhebung verteilt sind. Sie können über einen Kanal, der sich vor den Austrittsöffnungen verzweigt, oder über mehr als einen Kanal aus dem einen oder mehr als einen Flüssigkeitsreservoir gespeist werden. In einer besonders bevorzugten Ausführungsform weist die Wanne zwei oder mehr als zwei Erhebungen auf, und die eine oder mehr als eine Austrittsöffnung einer jeden Erhebung wird jeweils durch ein eigenes System gespeist, das wenigstens einen Kanal, wenigstens ein Zufuhrelement, wenigstens eine Pumpvorrichtung und wenigstens ein Flüssigkeitsreservoir umfasst. Es ist dann möglich, mehrere Ansätze mit immobilisiertem Reaktionspartner gleichzeitig mit verschiedenen Flüssigkeiten zu kontaktieren.

Zur Automatisierung und besseren Reproduzierbarkeit ist es vorteilhaft, wenn die wenigstens eine Flüssigkeit über eine geeignete Pumpvorrichtung aus dem Flüssigkeitsreservoir zur oberen Seite der Erhebung gepumpt wird. In einer bevorzugten Ausführungsform ist dazu eine oder mehr als eine Pumpe vorgesehen. Die Pumpvorrichtung ist bevorzugt so beschaffen, dass die wenigstens eine Flüssigkeit nicht nur eingeleitet, sondern auch abgesaugt werden kann. Beispielsweise können für das Einleiten und das Absaugen separate Pumpen vorgesehen sein oder eine Pumpe, deren Pumprichtung umkehrbar ist.

Als Flüssigkeitsreservoir kann jedes für die vorübergehende oder längere Aufbewahrung der einzuleitenden Flüssigkeit geeignete Gefäß dienen. Im Fall einer Waschlösung ist ein großvolumiges Vorratsgefäß sinnvoll, beispielsweise eine Glas- oder Plastikflasche. Für in geringeren Volumina zur Verfügung stehende Flüssigkeiten, beispielsweise flüssige Proben menschlichen oder tierischen Ursprungs, sind entsprechend kleiner dimensionierte Gefäße geeignet, beispielsweise Reagenzgläser, Mikrolitergefäße, Zentrifugiergefäße, Kapillaren o.ä. Bevorzugt kann eine Probe aus einem Transportbehälter oder aus einer zur Entnahme der Probe geeigneten Vorrichtung, beispielsweise einer Spritze, in die erfindungsgemäße Vorrichtung eingeleitet werden.

Besonders vorteilhaft ist es, wenn mehr als ein Flüssigkeitsreservoir derart mit dem Kanal verbunden ist, dass nacheinander oder gleichzeitig, mit frei wählbarem Mischungsverhältnis, zwei oder mehr als zwei verschiedene Flüssigkeiten eingeleitet werden können, beispielsweise eine Flüssigkeit mit einem Reaktanden, eine Waschlösung und/oder eine zweite Flüssigkeit. Als Zufuhrelement kommen Schläuche, Rohre und dergleichen in Betracht. Das Flüssigkeitsreservoir kann temperierbar sein, um reproduzierbare, für Nachweisverfahren optimale Bedingungen zu gewährleisten.

Die Halterungsvorrichtung dient dazu, die relative Position von Wanne und Objektträger zueinander zu bestimmen. Sie kann in die Vorrichtung integriert sein, bevorzugt unlösbar. In einer bevorzugten Ausführungsform weist die Wanne eine oder mehr als eine Objektträgerabstützfläche zum Aufnehmen des Objektträgers auf, die vorzugsweise im Randbereich des Objektträgers in ausreichendem Abstand zur Adhäsionsfläche ansetzt.

Bevorzugt können zwei an gegenüberliegenden Wänden der Wanne liegende oder befestigte Objektträgerabstützflächen, noch bevorzugter an den oberen Rändern der Wände liegende Objektträgerabstützflächen, das Einbringen des Objektträgers in die erfindungsgemäße Vorrichtung durch Auflegen auf zwei seiner gegenüberliegenden Ränder oder zwei seiner gegenüberliegenden Ecken ermöglichen. Alternativ kann der Objektträger an einer Seite lösbar über eine Klemme oder einen Greifer fixiert werden. Bevorzugt wird durch die Halterungsvorrichtung sichergestellt, dass sich der Objektträger nur entlang der Längsachse der Wanne bewegen kann. Ist eine solche Bewegung entlang der Längsachse erwünscht, so können Objektträger und Wanne mit einem Bewegungssystem, beispielsweise Rädern und Schienen, versehen sein. In einer weiteren Ausführungsform kann die Position des Objektträgers nach Einbringen in die Wanne fixiert sein, so dass der Objektträger unverrückbar aufliegt. Die Halterungsvorrichtung kann auch das vertikale Anheben oder Absenken des Objektträgers ermöglichen, optional auch einen oder mehr als einen Ventilator.

Erfindungsgemäß ist die Halterungsvorrichtung in einer Alternativlösung nicht unlösbar in die erfindungsgemäße Vorrichtung integriert, sondern als separates Teil ausgestattet, das wenigstens einen Objektträger, bevorzugt eine Vielzahl von Objektträgern, aufnehmen kann und dazu dient, diesen bzw. diese in die Vorrichtung einzubringen und für die Durchführung des erfindungsgemäßen Verfahrens zu positionieren. In einer bevorzugten Ausführungsform umfasst die Halterungsvorrichtung einen Rahmen, der für die Aufnahme und das Halten des Objektträgers ausgestattet ist, wobei sich die Adhäsionsfläche nach der Aufnahme auf der Seite des Objektträgers befindet, mit der er auf dem Rahmen aufliegt.

In einer Alternativlösung ist die erfindungsgemäße Vorrichtung bzw. die Halterungsvorrichtung für die Aufnahme und das Halten von mehr als einem Objektträger ausgestattet, vorzugsweise 3, 4, 6, 8, 10, 12, 14, 16, 20, 24, 28, 30, 32, 36, 40, 50, 60, 70, 80, 90, 100, 200, 300, 400, 500 oder 1000 Objektträger. Bei der Durchführung des Verfahrens muss gewährleistet sein, dass jede mit Flüssigkeit zu kontaktierende Adhäsionsfläche auf einem Objektträger in dafür geeigneter Weise zu einer Erhebung positioniert ist. Bevorzugt steht für jede mit Flüssigkeit zu kontaktierende Adhäsionsfläche eine Erhebung zur Verfügung. Es ist jedoch auch möglich, dass eine Erhebung die Flüssigkeit für mehr als eine Adhäsionsfläche bereitstellt oder eine Adhäsionsfläche von mehr als einer Erhebung mit Flüssigkeit kontaktiert wird.

Der Rahmen kann Mittel zum reversiblen oder irreversiblen Fixieren der Objektträger auf dem Rahmen umfassen, beispielsweise Vertiefungen, in die der Objektträger so eingebracht wird, dass seine Adhäsionsfläche in Richtung der Erhebungen, d. h. nach unten, zeigt, wenn die Halterungsvorrichtung von oben in die erfindungsgemäße Vorrichtung eingebracht wird. Der Objektträger kann durch bloßes Auflegen auf den Rahmen oder Fixieren, z. B. mit Klemmen o.ä. eingebracht werden.

Die genaue Position der Halterungsvorrichtung in der erfindungsgemäßen Vorrichtung kann durch die Form der ersteren, besonders durch das Profil ihrer Seitenwände, sofern vorhanden, festgelegt werden und ist so bemessen, dass der Abstand zwischen der oberen Seite der Erhebung und der Adhäsionsfläche die Durchführung des erfindungsgemäßen Verfahrens gestattet. Nach der Durchführung kann die Halterungsvorrichtung mit dem Objektträger wieder entnommen und, bei Bedarf, durch eine weitere Halterungsvorrichtung mit einem anderen Objektträger ersetzt werden. Auf diese Weise ist es gerade bei einer Vielzahl von abzuarbeitenden Objektträgern möglich, während des Verfahrens den jeweils nächsten Satz von Objektträgern parallel zum gerade laufenden Vorgang vorzubereiten und diesen dann einfach und zeitsparend in die Vorrichtung einzubringen.

Für viele Anwendungen ist es möglich, den Objektträger manuell in die Wanne einzubringen. Für eine erfindungsgemäße Vorrichtung, die für automatisierte Hochdurchsatzverfahren ausgelegt ist, ist jedoch bevorzugt, dass die Vorrichtung eine Transportvorrichtung aufweist, die den Objektträger oder die Halterungsvorrichtung mit dem Objektträger aus einer Lagerungseinheit oder Inkubationseinheit automatisch in die Wanne einbringt.

In einer bevorzugten Ausführungsform kann die Wanne in Anwesenheit oder Abwesenheit des Objektträgers druckfest verschlossen werden, so dass Flüssigkeiten durch Anlegen eines Unterdrucks abgezogen oder über Überdruck aus der Vorrichtung gedrängt werden. Besonders bevorzugt können Kanal, Zufuhrelement und, sofern vorhanden, Ablauföffnung und weitere Öffnungen druckfest verschlossen werden, beispielsweise über geeignete Hähne. Ein druckfester Verschluss erlaubt auch die Installation einer Gasmischstation, mit der innerhalb der Wanne eine Gasatmosphäre mit wählbarer Zusammensetzung erzeugt werden kann, beispielsweise eine Atmosphäre mit wenigstens einem Inertgas ausgewählt aus der Gruppe umfassend Stickstoff, Edelgase und Kohlendioxid, für gegenüber Sauerstoff empfindliche Substanzen. Für andere Anwendungen, bei denen die Anwesenheit von Luft nicht stört oder sogar erwünscht ist, beispielsweise, weil Luftbestandteile wie Sauerstoff für chemische Nachweisreaktionen erforderlich sind, kann die erfindungsgemäße Vorrichtung eine oder mehr als eine Öffnung zum Gasaustausch mit der Umgebung aufweisen.

Erfindungsgemäß ist es erforderlich, dass der Reaktionspartner immobilisiert ist. In einer bevorzugten Ausführungsform wird unter dem Begriff "Reaktionspartner", wie hierin verwendet, ein Molekül verstanden, dass von seiner chemischen Beschaffenheit geeignet ist, immobilisiert zu werden und gleichzeitig, auch im immobilisierten Zustand, eine spezifische Wechselwirkung mit einem in Flüssigkeit vorliegenden, gegebenen Reaktanden auszubilden. Beispielsweise kann es sich bei dem immobilisierten Reaktionspartner um ein Peptid oder Polypeptid, einen Naturstoff, ein künstliches Polymer oder eine Nukleinsäure, bevorzugt um DNA, oder eine Abwandlung oder Mischform davon handeln, ebenso um einen Molekülkomplex, beispielsweise um ein Protein aus mehreren Untereinheiten, oder um ein Substrat ausgewählt aus der Gruppe umfassend Dünnschnitte biologischer Gewebe, Zellausstriche von Säugetierzellen, Bakterien oder Ausstriche davon, Viren, Protozoen und Parasiten. Der Reaktionspartner kann in angereichter Form bis hin zu nahezu vollständiger Reinheit verwendet werden, aber auch in Form eines Gemischs, sofern dies die Ausbildung der Wechselwirkung zum Reaktanden nicht stark beeinträchtigt. Verfahren zur Aufreinigung typischer Reaktionspartner wie Nukleinsäuren, Naturstoffen oder Polypeptiden sind dem Fachmann bekannt. Es ist möglich, dass mehr als ein Reaktionsträger, beispielsweise wenigstens oder genau zwei, drei, vier oder 5 Reaktionspartner auf einer Adhäsionsfläche immobilisiert werden.

Bei der wenigstens einen Flüssigkeit kann es sich um eine beliebige Flüssigkeit handeln, die mit der Adhäsionsfläche auf dem Objektträger in Kontakt zu bringen ist. In einer bevorzugten Ausführungsform handelt es sich bei der Flüssigkeit um eine wenigstens einen Reaktanden enthaltende Flüssigkeit oder, noch bevorzugter um eine Flüssigkeit, bei der im Rahmen eines analytischen und/oder diagnostischen Verfahrens festgestellt werden soll, ob und optional in welcher Konzentration der Reaktand darin enthalten ist. In einer bevorzugten Ausführungsform ist unter dem Begriff "Reaktand", wie hierin verwendet, ein Molekül, ein Komplex, eine Struktur, eine Zelle oder dergleichen mit der Fähigkeit zur Bindung an den oder Reaktion mit dem immobilisierten Reaktionspartner zu verstehen. Beispielsweise kann es sich bei dem Reaktanden um ein Protein wie einen Antikörper oder ein Intercalin, um eine Nukleinsäure, um ein small molecule oder um einen Naturstoff handeln. Die Identifizierung und, falls erforderlich, Herstellung von Reaktanden für verschiedene Reaktionspartner ist dem Fachmann geläufig und im Stand der Technik beschrieben. Beispielsweise können Antikörper zu einem Polypeptidantigen durch Immunisierung eines Säugetieres hergestellt werden. Die Flüssigkeit kann auch eine Mischung umfassend mehr als einen Reaktanden erhalten.

In einer bevorzugten Ausführungsform handelt es sich bei der wenigstens einen oder zweiten Flüssigkeit um eine Probe menschlichen oder tierischen Ursprungs, die einen nachzuweisenden Reaktanden enthält, beispielsweise einen Metabolit, ein Protein, eine Nukleinsäure mit bestimmter Sequenz oder einen Antikörper, bevorzugt einen Autoantikörper. Besonders bevorzugt handelt es sich um eine Probe umfassend eine Körperflüssigkeit ausgewählt aus der Gruppe umfassend Serum, Urin, Liquor oder Speichel oder eine Verdünnung oder verarbeitete Form davon. Alternativ kann es sich um eine Probe aus Lebensmitteln, Getränken, Trink- oder Badewasser, Stuhl, Bodenmaterial o. ä. handeln. Bevorzugt wird die Probe nach der Gewinnung in geeigneter Weise verarbeitet, im Fall einer Blutprobe beispielsweise durch Abzentrifugieren der nichtlöslichen Bestandteile des Blutes, und/oder haltbar gemacht. Die Verarbeitung kann auch chemische Reaktionen und Derivatisierungen ausgehend von einer Probe als Edukt umfassen, beispielsweise die Durchführung einer Polymerase-Kettenreaktion zur Vervielfältigung einer in der Probe nachzuweisenden Nukleotidsequenz.

In einer weiteren bevorzugten Ausführungsform handelt es sich bei der wenigstens einen Flüssigkeit oder zweiten um eine Waschlösung. Waschlösungen dienen dazu, Teile der erfindungsgemäßen Vorrichtung wie die obere Seite der Erhebung, die Adhäsionsfläche ohne oder mit dem immobilisierten Reaktionspartner, den Kanal und/oder das Zufuhrelement von unerwünschten Substanzen zu befreien, beispielsweise von unerwünschten Bestandteilen flüssiger Proben, die für einen diagnostischen Nachweis nicht brauchbar sind, diesen sogar stören oder aus sonstigen Gründen unerwünscht sind. Der Fachmann ist in der Lage, geeignete Waschlösungen zu entwickeln und herzustellen, beispielsweise physiologische Kochsalzlösungen wie PBS-Puffer oder Puffer mit geringer Salzkonzentration, z. B. 50 mM Kaliumphosphat mit pH 7. Waschlösungen können auch oberflächenaktive Substanzen wie Alkohole, Detergenzien und Tenside zur Entfernung hydrophober Verunreinigungen enthalten. Insbesondere bei bevorstehenden längeren Lagerungen oder zur Desinfektion können auch antimikrobiell wirkende Substanzen wie Azid oder Ethanol eingesetzt werden.

In einer weiteren bevorzugten Ausführungsform handelt es sich bei der wenigstens einen oder der zweiten Flüssigkeit um eine Flüssigkeit, die für die Nachweisreaktion erforderliche Substanzen enthält. Handelt es sich bei dem immobilisierten Reaktionspartner und dem Reaktand beispielsweise um ein Antigen bzw. einen daran bindenden Antikörper, so kann nach Ausbildung des Antigen-Antikörper-Komplexes eine Lösung mit einem zweiten, enzym- oder fluoreszenzmarkierten Antikörper in die erfindungsgemäße Vorrichtung eingeleitet werden, so dass auf dem Objektträger ein nachweisbarer Komplex immobilisiert vorliegt. In einer bevorzugten Ausführungsform umfasst die wenigstens eine oder zweite Flüssigkeit einen Farbstoff, mit dem ein an der Adhäsionsfläche immobilisierter Gewebeschnitt oder an der Adhäsionsfläche immobilisierte prokaryontische oder eukaryontische Zellen oder Präparationen angefärbt werden.

Die Immobilisierung des Reaktionspartners kann reversibel oder irreversibel sein, solange sie für die Durchführung des erfindungsgemäßen Verfahrens ausreichend beständig ist. Bevorzugt ist der Reaktionspartner über wenigstens eine kovalente chemische Bindung immobilisiert. Dem Fachmann sind zahlreiche Verfahren zur Immobilisierung von Molekülen auf Oberflächen unterschiedlichster Beschaffenheit bekannt. Beispielsweise können Proteine über chemische Aktivierung an Kohlenhydrat-, Glas- oder Polymeroberflächen immobilisiert werden, wie z. B. in der US20100056764 oder in der US 5137765 beschrieben ist. Alternativ kann eine Befestigung über eine ausreichend starke ionische oder hydrophobe Wechselwirkung erreicht werden. Beispielsweise kann ein Protein mit negativer Ladung an eine anorganische Oberfläche mit positiver Ladung mit hoher Affinität binden, wie es z. B. in US 4206286 beschrieben ist.

Die erfindungsgemäße Vorrichtung ermöglicht es, dass die Flüssigkeit aus dem Flüssigkeitsreservoir über ein Zufuhrelement mittels einer Pumpvorrichtung durch die Austrittsöffnung an die obere Seite der Erhebung gelangt, um mit der Adhäsionsfläche in Kontakt zu treten. Die Dauer des Kontakts und die Bedingungen, unter denen dieser stattfindet, können je nach Natur und Funktion der Flüssigkeit variiert werden. Handelt es sich bei der Flüssigkeit um eine Flüssigkeit mit darin gelösten Reaktanden, die mit dem immobilisierten Reaktionspartner eine Bindung oder Reaktion eingehen sollen, so muss der Kontakt lange genug andauern, damit eine ausreichende Zahl von Molekülen des Reaktanden mit dem immobilisierten Reaktionspartner wechselwirken und beispielsweise einen nachweisbaren Komplex bilden kann. Beispielsweise kann der Kontakt wenigstens 10 Sekunden, 30 Sekunden, eine, zwei, fünf, zehn, 15, 30, 60, 120 oder 180 Minuten andauern. Handelt es sich bei der Flüssigkeit um eine Waschflüssigkeit, so kann ein kurzer Kontakt genügen.

Die Flüssigkeit kann je nach Bedarf kontinuierlich oder diskontinuierlich, d. h. in Schüben, nach denen der Pumpvorgang unterbrochen wird, in die erfindungsgemäße Vorrichtung eingeleitet werden. Handelt es sich um eine Flüssigkeit, von der nur geringe Volumina zur Verfügung stehen, beispielsweise um eine Blutprobe menschlichen oder tierischen Ursprungs, so kann die verfügbare Menge eingeleitet und die Zufuhr unterbrochen werden, nachdem die Adhäsionsfläche ausreichend benetzt ist. Während der folgenden Inkubation kann dann die Wechselwirkung zwischen Reaktionspartner und Reaktand stattfinden. Handelt es sich bei der Flüssigkeit hingegen um eine Waschlösung, so kann sie in größeren Mengen kontinuierlich eingeleitet werden, um die Adhäsionsfläche des Objektträgers gründlich zu waschen.

Die auf die obere Seite der Erhebung gelangte Flüssigkeit sammelt sich an, solange ihr Volumen nicht das Volumen übersteigt, das zwischen Adhäsionsfläche und oberer Seite der Erhebung festgehalten werden kann, z. B. durch hydrophile Wechselwirkungen zwischen Flüssigkeit und Adhäsionsfläche sowie oberer Seite der Erhebung. In einer bevorzugten Ausführungsform sind die Austrittsöffnung auf der oberen Seite der Erhebung, die obere Seite der Erhebung und die Adhäsionsfläche derart ausgestaltet, dass die wenigstens eine Flüssigkeit tangential am immobilisierten Reaktionspartner entlang strömt, wenn sie in die Vorrichtung eingeleitet wird. Wird mehr Flüssigkeit eingeleitet als zwischen Adhäsionsfläche und oberer Seite der Erhebung festgehalten werden kann, so läuft diese auf der Erhebung nach den Seiten hin auf den Boden der Wanne und kann über eine Ablauföffnung, sofern vorhanden, ablaufen. Mit der Adhäsionsfläche in Kontakt stehende Flüssigkeit kann auch durch Verdunsten, optional beschleunigt durch Anlegen von Unterdruck an die Wanne, sofern sie druckdicht verschließbar ist, oder durch Verdrängung über eine weitere eingeleitete Flüssigkeit oder durch Einblasen eines Gases entfernt werden.

Mit der Adhäsionsfläche in Kontakt stehende Flüssigkeit kann auch aktiv über die Austrittsöffnung und den darin mündenden Kanal abgesaugt werden. Die Flüssigkeit kann dann zurück in das Flüssigkeitsreservoir oder, besonders bevorzugt, über einen eigenen Ablauf am Zufuhrelement aus der Vorrichtung entnommen und verworfen, weiter verwendet oder gelagert werden.

In einer bevorzugten Ausführungsform weist die Adhäsionsfläche auf dem Objektträger eine längliche Form auf, wie sie in anderem Zusammenhang im Stand der Technik beschrieben ist, beispielsweise in der EP 2 191 893. Bei einer Wanne mit rechteckigem Boden kann die Adhäsionsfläche entlang oder entgegen der Längsachse der Wanne angeordnet sein. In einer besonders bevorzugten Ausführungsform ist die Erhebung, zu einer solchen länglichen Form der Adhäsionsfläche passend, derart als Rinne ausgestaltet, dass die längliche Adhäsionsfläche am Objektträger ihr gegenüber liegt und die wenigstens eine Flüssigkeit beim Einleiten über das Zufuhrelement in Richtung der Austrittsöffnung mit dem an der Adhäsionsfläche immobilisierten Reaktionspartner in Kontakt tritt. Optional weist die als Rinne ausgestaltete Erhebung wenigstens zwei Kanäle, jeweils mit einer Austrittsöffnung, auf, wobei einer der Kanäle zum Zuführen der Flüssigkeit und der andere zum Abpumpen der Flüssigkeit dienen kann; besonders bevorzugt liegt dann an jedem der beiden Enden der Rinne eine Austrittsöffnung. Alternativ kann die Rinne auch eine Reihe von Austrittsöffnungen aufweisen, die über die Längsachse der Rinne gleichmäßig verteilt sind.

Die erfindungsgemäße Vorrichtung kann zur Durchführung eines Verfahrens verwendet werden, das das Kontaktieren eines immobilisierten Reaktionspartners mit wenigstens einer Flüssigkeit umfasst.

Je nach Problemstellung und Eigenschaften des immobilisierten Reaktionspartners und des Reaktanden können einzelne, aber auch sämtliche Schritte des Nachweisverfahrens mit Hilfe der erfindungsgemäßen Vorrichtung durchgeführt werden, bei Bedarf unter Verwendung verschiedener Flüssigkeiten in beliebiger Reihenfolge.

In jedem Fall ist es erforderlich, die erfindungsgemäße Vorrichtung umfassend den Objektträger mit dem immobilisierten Reaktionspartner an der Adhäsionsfläche bereitzustellen. Die Immobilisierung kann dabei in einer bevorzugten Ausführungsform bereits in der erfindungsgemäßen Vorrichtung stattfinden. Der Objektträger mit der Adhäsionsfläche, aber ohne immobilisierten Reaktionspartner, wird dazu in die erfindungsgemäße Vorrichtung eingebracht. Anschließend wird über den Kanal in der Erhebung eine Flüssigkeit in die Vorrichtung eingeleitet, die den zu immobilisierenden Reaktionspartner und optional weitere für die Immobilisierung erforderliche Substanzen umfasst, bis diese Flüssigkeit mit der Adhäsionsfläche in Kontakt steht, so dass es zur Immobilisierung des Reaktionspartners kommt. Die Flüssigkeit wird anschließend entfernt, beispielsweise durch Absaugen oder durch Verdrängung durch Einleitung einer anderen Flüssigkeit, beispielsweise einer Waschlösung. Alternativ kann der Reaktionsträger außerhalb der erfindungsgemäßen Vorrichtung immobilisiert werden, gefolgt vom Einbringen des Objektträgers in die erfindungsgemäße Vorrichtung.

Sofern es die chemischen Eigenschaften der Adhäsionsfläche und des zu immobilisierenden Reaktionspartners erfordern, kann die Adhäsionsfläche vor der Immobilisierung noch mit einer weiteren Flüssigkeit kontaktiert werden, die wenigstens eine aktivierende Substanz umfasst, die auf der Adhäsionsfläche vorhandene chemische Gruppen derart chemisch aktiviert, dass die nachfolgende Immobilisierung effizienter oder überhaupt erst in ausreichendem Ausmaß abläuft.

Nach den beiden vorgenannten Schritten, genauer der eigentlichen Immobilisierung des Reaktionspartners sowie einer optional vorher durchzuführenden chemischen Aktivierung des Adhäsionsfläche, wie auch nach anderen Schritten, bei denen in die erfindungsgemäße Vorrichtung eine Flüssigkeit mit Substanzen eingeleitet wird, die mit der Adhäsionsfläche und daran immobilisierten Molekülen wechselwirken können, ist es empfehlenswert, einen Waschschritt durchzuführen. Im einfachsten Fall wird dazu eine Waschlösung in die erfindungsgemäße Vorrichtung eingeleitet, bis die Adhäsionsfläche mit dem immobilisierten Reaktionspartner und/oder alle Komponenten der erfindungsgemäßen Vorrichtung, die mit eingeleiteten Flüssigkeiten in Berührung kommen, ausreichend gereinigt sind. Auch vor der erstmaligen Verwendung neuer Geräte oder Teile davon, insbesondere vor der erstmaligen Verwendung von Objektträgern und/oder vorgefertigten Substraten, oder zur Vorbereitung der erfindungsgemäßen Vorrichtung für einen neuen Versuch, kann ein Waschschritt empfehlenswert sein, um Flüssigkeiten mit Substanzen zu entfernen, die aus vorherigen Versuchen stammen oder für die Lagerung und/oder den Transport neuer Geräte oder Teile davon verwendet werden und bei der eigentlichen Anwendung stören könnten.

Das Einleiten der Waschlösung kann so lange andauern, bis die erfindungsgemäße Vorrichtung vollständig von Verunreinigungen befreit ist. In einer bevorzugten Ausführungsform wird unter dem Begriff "vollständig von Verunreinigungen befreit", wie hierin verwendet, verstanden, dass bei weiterem Einleiten von Waschlösung in die Vorrichtung in der aus der Vorrichtung nach dem Waschschritt austretenden Waschlösung keine Verunreinigungen mehr nachweisbar sind.

Soll der Reaktionspartner nicht innerhalb der erfindungsgemäßen Vorrichtung immobilisiert werden, so kann dies vor dem Einbringen des Objektträgers in die erfindungsgemäße Vorrichtung geschehen. Das ist insbesondere dann empfehlenswert, wenn der zu immobilisierende Reaktionspartner in geringer Menge und/oder in einem kleinen Volumen vorliegt. Das Einleiten in die erfindungsgemäße Vorrichtung kann in diesem Fall zu einem hohen Verlust an Reaktionspartner führen, beispielsweise wenn er von Oberflächen, beispielsweise im Kanal, unspezifisch adsorbiert oder stark verdünnt wird und nur ein geringer Teil davon bis zur Adhäsionsfläche gelangt und dort immobilisiert werden kann. Nach der Immobilisierung wird der Objektträger in die Halterungsvorrichtung der erfindungsgemäßen Vorrichtung eingebracht und kann darin mit beliebigen weiteren Flüssigkeiten kontaktiert werden, beispielsweise mit einer Flüssigkeit umfassend einen Reaktanden. Auch nach einer nicht innerhalb der erfindungsgemäßen Vorrichtung durchgeführten Immobilisierung kann der Objektträger gewaschen werden. Dies kann noch außerhalb der erfindungsgemäßen Vorrichtung geschehen oder innerhalb der Vorrichtung durch Einleiten von Waschlösung.

Auch das Kontaktieren des immobilisierten Reaktionspartners mit dem Reaktand kann innerhalb oder außerhalb der Vorrichtung erfolgen. Wird der immobilisierte Reaktionspartner innerhalb der erfindungsgemäßen Vorrichtung immobilisiert, soll das Kontaktieren mit dem Reaktanden aber außerhalb erfolgen, so muss der Objektträger zu diesem Zweck entnommen werden. Wiederum ist ein Kontaktieren außerhalb der Vorrichtung besonders dann empfehlenswert, wenn der Reaktand in geringer Menge und/oder in einem kleinen Volumen vorliegt, beispielsweise, wenn der Reaktand in einer Blutprobe von einem Säugling zu bestimmen ist.

Eine weitere besondere Stärke der erfindungsgemäßen Vorrichtung und des damit ausgeführten erfindungsgemäßen Verfahrens besteht darin, dass mehrere Flüssigkeiten mit jeweils einer für eine Nachweisreaktion benötigten Komponente nacheinander eingeleitet werden können, ohne dass zusätzlicher manueller Arbeitsaufwand entsteht. Bei einer manuellen Fahrweise neigen viele Anwender in der Praxis dazu, die verschiedenen Komponenten nicht nacheinander über separate Flüssigkeiten reagieren zu lassen, sondern in weniger Schritten, bevorzugt einem einzigen Schritt, mit einem Gemisch der Komponenten. Das Einleiten der Reagenzien nacheinander führt besonders im Bereich der indirekten Immunfluoreszenz zu qualitativ besseren Resultaten. Sollen beispielsweise in einer Probe Antikörper mehrerer Immunglobulinklassen (IgA, IgG und IgM) bestimmt werden, dann führt eine sequentielle Inkubation mit fluoreszenzmarkierten sekundären Antikörpern gegen IgA, IgG und IgM im allgemeinen zu eindeutigeren Reaktionen als es der Fall ist, wenn mit einem Reagenziengemisch inkubiert wird. Eine weitere besondere Stärke der vorliegenden Erfindung besteht darin, dass eine mit der Adhäsionsfläche in Kontakt stehende Flüssigkeit über den Kanal in der Erhebung nicht nur eingeleitet, sondern auch wieder durch Absaugen entfernt werden kann. Eine Flüssigkeit, die nach dem Kontaktieren mit der Adhäsionsfläche erneut verwendet werden soll, kann auf diese Weise separat für jede Erhebung zurückgewonnen und, sofern erforderlich, gelagert und/oder für andere Anwendungen, z. B. andere analytische Verfahren verwendet werden, ohne dass es zu einer Vermischung mit Flüssigkeiten aus anderen Ansätzen kommen könnte.

Die Option, die Flüssigkeit abzusaugen, ermöglicht es auch, chemische Nachweisreaktionen, insbesondere chromogene Farbreaktionen, beispielsweise zum Nachweis eines enzymatisch aktiven Reaktanden oder eines enzymmarkierten zweiten Antikörpers, in der erfindungsgemäßen Vorrichtung durchzuführen und anschließend auszuwerten. Zu diesem Zweck kann nach Ausbildung des Komplexes umfassend den immobilisierten Reaktionspartner und den enzymatisch aktiven Reaktand oder den enzymmarkierten zweiten Antikörper eine Flüssigkeit umfassend ein chromogenes Substrat in die erfindungsgemäße Vorrichtung eingeleitet werden, bis sie in Kontakt mit der Adhäsionsfläche steht und das Substrat enzymatisch umgesetzt werden kann. Nach einer geeigneten Inkubationsdauer kann sie abgesaugt und in einer Detektionsvorrichtung untersucht werden, im Falle des chromogenen Substrates beispielsweise in einem UV/vis-Spektrometer. In einer besonders bevorzugten Ausführungsform ist die erfindungsgemäße Vorrichtung selbst mit einer solchen Detektionsvorrichtung ausgestattet. Beispielsweise kann der mit Flüssigkeit gefüllte Zwischenraum zwischen der Adhäsionsfläche und der oberen Seite der Erhebung von der einen Seite mit elektromagnetischer Strahlung geeigneter Wellenlänge bestrahlt und die Absorption der Strahlung in der Flüssigkeit auf der anderen Seite der Erhebung gemessen werden. Alternativ kann die abgesaugte Flüssigkeit in eine an den Kanal oder das Zufuhrelement angeschlossene Detektionsvorrichtung geleitet und dort gemessen werden.

In einer besonders bevorzugten Ausführungsform wird der Objektträger in der oder außerhalb der erfindungsgemäßen Vorrichtung nach dem Einleiten der ersten, zweiten oder weiteren Flüssigkeit oder der Waschlösung einem weiteren Schritt f) Trocknen der Adhäsionsfläche unterzogen. Das Trocknen erfolgt bevorzugt dadurch, dass der Objektträger mit Hilfe der Halterungsvorrichtung verschoben wird, bevorzugt entlang der Längsachse der Wanne, bis die Adhäsionsfläche von dem Luftstrahl wenigstens einer optional zur Ausstattung der erfindungsgemäßen Vorrichtung gehörenden Luftdüse getrocknet wird. Bevorzugt wird Preßluft durch die Luftdüse geleitet.

In einer weiteren, besonders bevorzugten Ausführungsform wird der Objektträger in der oder außerhalb der erfindungsgemäßen Vorrichtung nach dem Einleiten der ersten, zweiten oder weiteren Flüssigkeit oder der Waschlösung oder dem weiteren Schritt f) in geeigneter Weise für die Auswertung vorbereitet, beispielsweise durch Aufbringen von Eindeckmedium, beispielsweise mit Glycerin versetzte Phosphat-gepufferte Kochsalzlösung, und Auflegen eines Deckglases.

In einer bevorzugten Ausführungsform wird das erfindungsgemäße Verfahren zur Durchführung eines ELISA-Tests genutzt, mit dem Autoantikörper in einer menschlichen Blutprobe nachgewiesen werden können. Zu diesem Zweck wird als immobilisierter Reaktionspartner ein vom Autoantikörper erkanntes Antigen, beispielsweise ein Polypeptid, in gereinigter Form oder in Form einer Zelle oder eines Gewebes an der Adhäsionsfläche des Objektträgers immobilisiert. Die Adhäsionsfläche wird anschließend gewaschen und mit der zu untersuchenden menschlichen Blutprobe kontaktiert, die den Autoantikörper (Reaktand) enthalten kann, gefolgt von einem weiteren Waschschritt. Danach wird die Adhäsionsfläche mit dem immobilisierten Antigen mit einer Lösung kontaktiert, die einen zweiten Antikörper enthält, der sich an den Autoantikörper bindet und zum Nachweis des Komplexes aus Antigen, Autoantikörper und zweitem Antikörper eine Markierung aufweist, im Falle des ELISA ein aktives Enzym, beispielsweise eine Peroxidase. Schließlich kann der Objektträger der erfindungsgemäßen Apparatur entnommen werden und mit einem chromogenen Substrat der Peroxidase behandelt werden. Eine positive Farbreaktion zeigt das Vorhandensein des Autoantikörpers an.

Dem Fachmann sind zahlreiche Varianten des ELISA-Tests bekannt, die ebenfalls im Rahmen des erfindungsgemäßen Verfahrens durchgeführt werden können. Beispielsweise kann für den Sandwich-ELISA zunächst ein erster Antikörper (Reaktionspartner) auf der Adhäsionsfläche immobilisiert werden, der ein Antigen (Reaktand) erkennt, das in einer menschlichen Probe nachzuweisen ist. Der Nachweis erfolgt dann über einen zweiten Antikörper, der ebenfalls an den Reaktand bindet.

Alternativ kann das erfindungsgemäße Verfahren für den immunfluoresenzbasierten Nachweis von Autoantikörpern eingesetzt werden. Die Durchführung erfolgt wie beim ELISA-Test, abgesehen davon, dass der zweite Antikörper mit einem fluoreszierenden Molekül markiert ist, beispielsweise mit Fluoreszein. Der Komplex aus Antigen, Autoantikörper und zweitem Antikörper wird in diesem Fall mit Hilfe eines Fluoreszenzmikroskops nachgewiesen.

Alternativ kann das erfindungsgemäße Verfahren zum Nachweis von spezifischen Nukleinsäuresequenzen verwendet werden, beispielsweise von PCR-Produkten, die ausgehend von einer menschlichen Probe mit einer nachzuweisenden Nukleinsäure als Template gewonnen werden. In diesem Fall wird als immobilisierter Reaktionspartner eine Nukleinsäure verwendet, die spezifisch an die nachzuweisende Nukleinsäuresequenz (Reaktand) bindet. Das Kontaktieren von immobilisiertem Reaktionspartner und Reaktand findet dann unter Bedingungen statt, die eine spezifische Bindung zulassen. Derartige Bedingungen kann der Fachmann routinemäßig auffinden. Für den Nachweis des Komplexes aus immobilisiertem Reaktionspartner und Reaktand stehen ebenfalls routinemäßig anwendbare Methoden zur Verfügung, beispielsweise der Nachweis über Fluoreszenz.

Im Folgenden wird die Erfindung unter Bezugnahme auf die Figuren anhand von Ausführungsbeispielen erläutert. Die beschriebenen Ausführungsformen sind in jeder Hinsicht lediglich beispielhaft und nicht als einschränkend zu verstehen, und verschiedene Kombinationen der angeführten Merkmale sind vom Umfang der Erfindung umfasst.

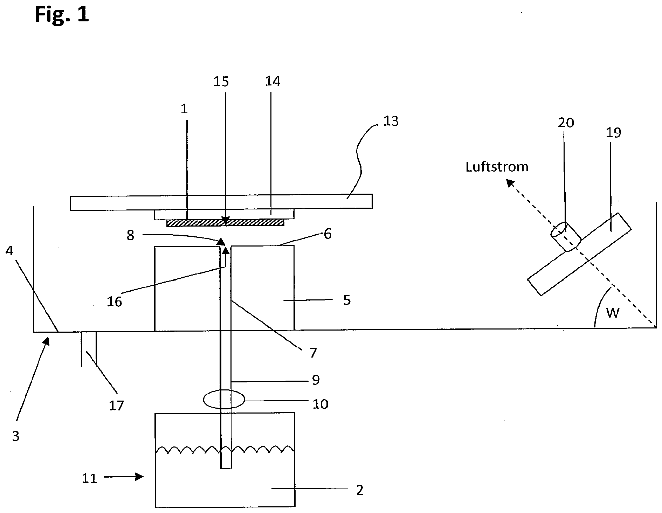

**Fig. 1** zeigt eine erfindungsgemäße Vorrichtung von der Seite, entgegen der Längsachse der Vorrichtung,

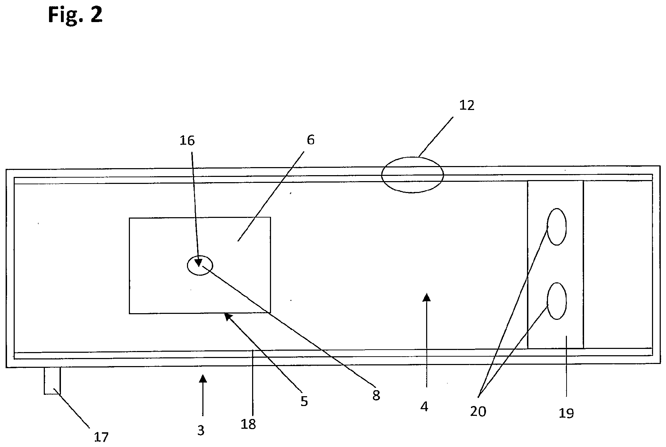

**Fig. 2** zeigt die erfindungsgemäße Vorrichtung von oben, und

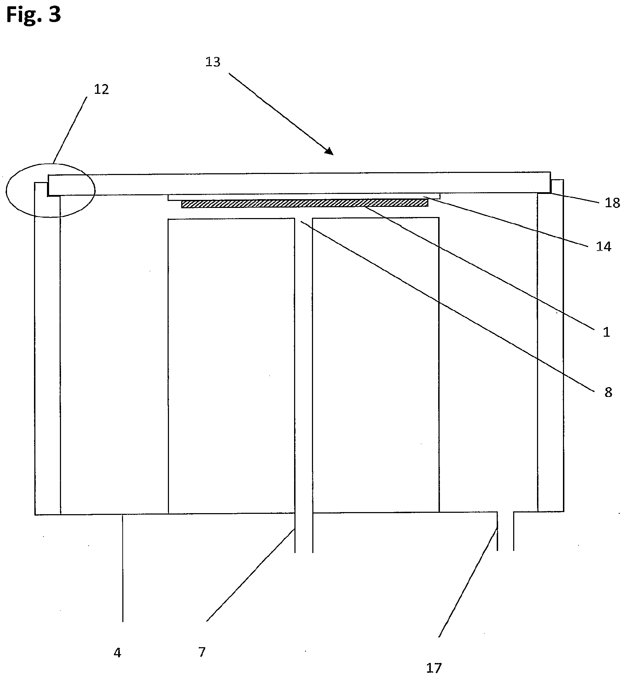

**Fig. 3** zeigt die erfindungsgemäße Vorrichtung von vorne, entlang der Längsachse der Vorrichtung,

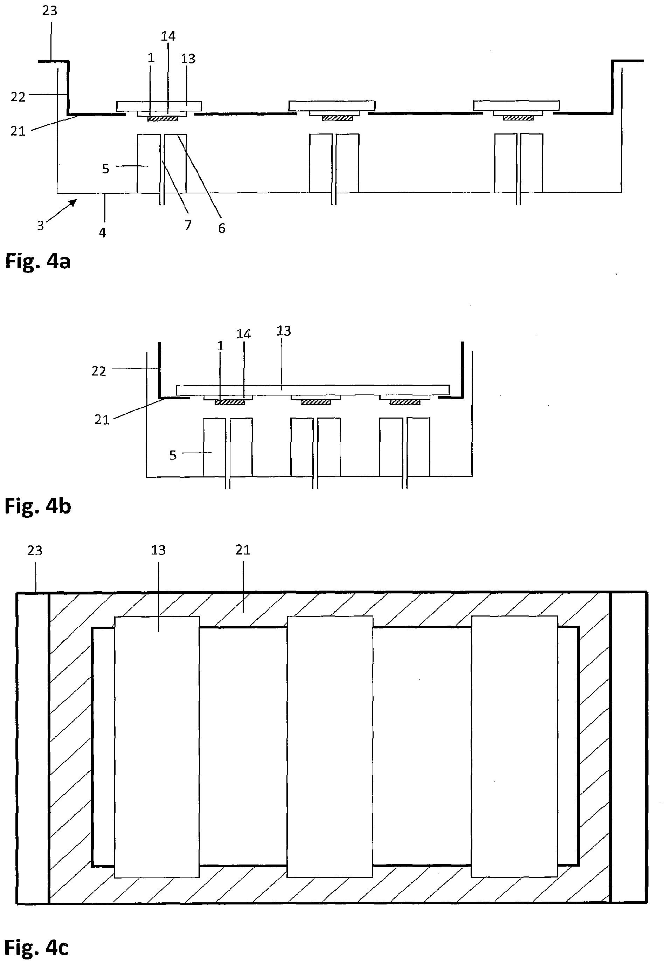

**Figs. 4a, 4b** und **4c** zeigen die erfindungsgemäße Vorrichtung mit einer Halterungsvorrichtung von der Seite, entgegen der Längsachse der Vorrichtung **(****Fig. 4a****),** im Querschnitt, entlang der Längsachse der Vorrichtung **(****Fig. 4b****),** und von oben **(****Fig. 4c****),**
wobei einige Details zwecks Übersichtlichkeit nicht dargestellt sind.

Wie aus diesen Figuren ersichtlich ist, kann die erfindungsgemäße Lehre ausgeführt werden unter Verwendung einer Vorrichtung zum Kontaktieren wenigstens eines immobilisierten Reaktionspartners (1) mit wenigstens einer Flüssigkeit (2), umfassend eine Wanne (3) mit einem Boden (4), aus dem wenigstens eine Erhebung (5) mit einer oberen Seite (6) in den Innenraum der Wanne (3) ragt, wobei die wenigstens eine Erhebung (5) einen Kanal (7) aufweist, der senkrecht zum Boden (4) der Wanne (3) verläuft und auf der oberen Seite (6) der Erhebung (5) in wenigstens eine Austrittsöffnung (8) mündet, wobei der Kanal (7) über ein Zufuhrelement (9) mittels einer Pumpvorrichtung (10) aus wenigstens einem Flüssigkeitsreservoir (11) gespeist wird, das die wenigstens eine Flüssigkeit (2) enthält, eine Halterungsvorrichtung (12), die einen Objektträger (13) aufweisend wenigstens eine Adhäsionsfläche (14) mit dem wenigstens einen immobilisierten Reagenz (1), lösbar derart hält, dass die Adhäsionsfläche (14) dem Innenraum der Wanne (3) zugewandt ist, wobei die obere Seite (6) der Erhebung (5) und die Adhäsionsfläche (14) derart relativ zueinander positioniert sind, dass aus der Austrittsöffnung (8) austretende Flüssigkeit mit dem immobilisierten Reagenz in Kontakt steht.

Bevorzugt weist die Adhäsionsfläche (14) einen Mittelpunkt (15) auf und die obere Seite (6) der Erhebung (5) weist einen Mittelpunkt (16) auf, wobei die obere Seite (6) der Erhebung (5) und die Adhäsionsfläche (14) derart positioniert sind, dass der Mittelpunkt (15) der Adhäsionsfläche (14) und der Mittelpunkt (16) der oberen Seite der Erhebung (5) auf einer senkrecht zur Längsachse der Wanne (3) verlaufenden Geraden liegen.

Bevorzugt weist die Wanne (3) wenigstens eine Ablauföffnung (17) auf.

Bevorzugt gestattet die Halterungsvorrichtung (12) eine Verschiebung des Objektträgers (13) entlang der Längsachse der Wanne (3), besonders bevorzugt über eine an den oberen Rändern der Wanne (3) angebrachte Rinne (18) oder Schiene.

Bevorzugt ist die Wanne weiterhin mit wenigstens einer Luftdüse (20) auf einem der Positionierung der Luftdüse dienenden Träger (19) zum Trocknen der Adhäsionsfläche (14) ausgestattet,
wobei besonders bevorzugt der Luftstrahl aus der wenigstens einen Luftdüse (20) mit der durch den Boden (4) der Wanne (3) festgelegten Ebene einen Winkel (W) von weniger als 90 Grad, bevorzugt 20 bis 70 Grad bildet.

Bevorzugt ist die erfindungsgemäße Vorrichtung mit einer Halterungsvorrichtung ausgestattet, die einen Rahmen (21), wenigstens zwei sich an gegenüberliegenden Seiten des Rahmens erhebende Seitenwände (22) und einen Griff (23).

### Bezugszeichenliste:

(1) Reagenz
(2) Flüssigkeit
(3) Wanne
(4) Boden
(5) Erhebung
(6) Obere Seite der Erhebung
(7) Kanal
(8) Austrittsöffnung
(9) Zufuhrelement
(10) Pumpvorrichtung
(11) Flüssigkeitsreservoir
(12) Halterungsvorrichtung
(13) Objektträger
(14) Adhäsionsfläche
(15) Mittelpunkt der Adhäsionsfläche
(16) Mittelpunkt der Erhebung
(17) Ablauföffnung
(18) Rinne
(19) Träger
(20) Luftdüse
(21) Rahmen
(22) Seitenende der Halterungsvorrichtung
(23) Griff

## Patentansprüche

1. Vorrichtung zum Kontaktieren eines immobilisierten Reaktionspartners mit wenigstens einer Flüssigkeit, umfassend
eine Wanne (3) mit einem Boden (4), aus dem wenigstens eine Erhebung (5) mit einer oberen Seite (6) in den Innenraum der Wanne (3) ragt,
wobei die wenigstens eine Erhebung (5) einen Kanal (7) aufweist, der vom Boden (4) der Wanne (3) zur oberen Seite der Erhebung (6), bevorzugt senkrecht zum Boden (4) der Wanne (3), verläuft und auf der oberen Seite der Erhebung (6) in wenigstens eine Austrittsöffnung (8) mündet,
wobei der Kanal (7) über ein Zufuhrelement (9) mittels einer Pumpvorrichtung (10) aus wenigstens einem Flüssigkeitsreservoir (11) gespeist wird, das die wenigstens eine Flüssigkeit (2) enthält,
eine Halterungsvorrichtung (12), die eingerichtet ist, um wenigstens einen Objektträger (13) aufweisend wenigstens eine Adhäsionsfläche (14) mit dem immobilisierten Reaktionspartner lösbar derart zu halten, dass die Adhäsionsfläche (14) dem Innenraum der Wanne (3) zugewandt ist, und dass die obere Seite der Erhebung (6) und die Adhäsionsfläche (14) derart zueinander positioniert sind, dass aus der Austrittsöffnung (8) austretende Flüssigkeit (2) mit dem immobilisierten Reagenz in Kontakt steht,
wobei die Vorrichtung wenigstens einen in die Halterungsvorrichtung (12) eingebrachten Objektträger (13) umfasst,
wobei die obere Seite der Erhebung (6) und die Adhäsionsfläche (14) parallel zueinander angeordnet sind,
und wobei der Objektträger (13) wenigstens zwei voneinander abgegrenzte Adhäsionsflächen (14) aufweist oder die Halterungsvorrichtung (12) als separates Teil ausgestattet ist, das für die Aufnahme und das Halten von wenigstens zwei Objektträgern (13) ausgestattet ist,
charakterisiert dadurch, dass die Vorrichtung in der einen Wanne (3) zwei oder mehr Erhebungen (6) und entsprechend zwei oder mehr korrespondierende Adhäsionsflächen (14) aufweist,
und dass diese derart gestaltet sind und sich in einem ausreichendem Abstand voneinander befinden, dass die durch die Erhebungen (6) eingeleitete Flüssigkeit (2) nicht mit Adhäsionsflächen (14) in Berührung kommen, die benachbarten Erhebungen gegenüber liegen.

2. Vorrichtung nach Anspruch 1,
wobei der Kanal (7) zusätzlich aus einem weiteren Flüssigkeitsreservoir (11) gespeist wird, das eine zweite Flüssigkeit enthält.

3. Vorrichtung nach einem der Ansprüche 1 bis 2,
wobei der Kanal (7) auf der oberen Seite der Erhebung (6) in mehr als eine Austrittsöffnung (8) mündet.

4. Vorrichtung nach einem der Ansprüche 1 bis 3,
wobei die Adhäsionsfläche (14) einen Mittelpunkt (15) aufweist und die obere Seite der Erhebung (6) einen Mittelpunkt (16) aufweist,
und wobei sich Mittelpunkt der Adhäsionsfläche (15) und der Mittelpunkt der oberen Seite der Erhebung (16) gegenüber liegen.

5. Vorrichtung nach einem der Ansprüche 1 bis 4,
wobei die Wanne (3) wenigstens eine Ablauföffnung (17) aufweist.

6. Vorrichtung nach einem der Ansprüche 1 bis 5,
wobei die Halterungsvorrichtung (12) eine Verschiebung des Objektträgers (13) gestattet, bevorzugt über wenigstens eine an den oberen Rändern der Wanne (3) angebrachte Rinne (18) oder Schiene.

7. Vorrichtung nach Anspruch 6, wobei die Wanne (3) weiterhin mit wenigstens einer Luftdüse (20) zum Trocknen der Adhäsionsfläche (14) ausgestattet ist.

8. Vorrichtung nach einem der Ansprüche 1 bis 7,
wobei die Pumpvorrichtung (10) gestattet, dass irgendeine Flüssigkeit (2), die mit dem immobilisierten Reaktionspartner in Kontakt steht, aus Richtung der Austrittsöffnung (8) abgesaugt wird.

9. Vorrichtung, nach einem der Ansprüche 1 bis 8,
weiter umfassend eine Transportvorrichtung, die das Einbringen des Objektträgers (13) in die Vorrichtung gestattet.

10. Vorrichtung nach einem der Ansprüche 1 bis 9, wobei die Austrittsöffnung (8) auf der oberen Seite der Erhebung (6), die obere Seite der Erhebung (6) und die Adhäsionsfläche (14) derart ausgestaltet sind, dass die wenigstens eine Flüssigkeit (2) tangential am immobilisierten Reaktionspartner entlang strömt, wenn sie in die Vorrichtung eingeleitet wird.

11. Vorrichtung nach einem der Ansprüche 1 bis 10,
wobei die Halterungsvorrichtung (12) einen Rahmen (21) umfasst, der für die Aufnahme und das Halten wenigstens eines Objektträgers (13), bevorzugt einer Vielzahl von Objektträgern (13), ausgestattet ist, wobei sich die Adhäsionsfläche (14) nach der Aufnahme auf der Seite des Objektträgers (13) befindet, mit der er auf dem Rahmen (21) aufliegt.

12. Vorrichtung nach einem der Ansprüche 1 bis 11, wobei die relative Position von Wanne (3) und Objektträgern (13) durch die Halterungsvorrichtung (12) bestimmt ist.

13. Vorrichtung nach einem der Ansprüche 1 bis 11, wobei die Vorrichtung so ausgestaltet ist, dass mehr Flüssigkeit, als zwischen Adhäsionsfläche (14) und oberer Seite der Erhebung (6) festgehalten werden kann, nach den Seiten der Erhebung hin auf dem Boden (4) der Wanne (3) ablaufen kann .

14. Vorrichtung nach einem der Ansprüche 1 bis 13, wobei der Rahmen (21) wenigstens eine Seitenwand aufweist, die bevorzugt mit einem Griff (23) ausgestattet ist.

15. Vorrichtung nach Anspruch 14, wobei die Seitenwand des Rahmens (21) ein Profil aufweist, das zum Positionieren der Halterungsvorrichtung (12) und damit der Adhäsionsfläche (14) in der Vorrichtung geeignet ist.

16. Verfahren zum Kontaktieren eines immobilisierten Reaktionspartners mit wenigstens einer Flüssigkeit, umfassend die Schritte
a) Bereitstellen der Vorrichtung nach einem der Ansprüche 1 bis 15 umfassend wenigstens einen Objektträger (13) mit immobilisiertem Reaktionspartner,
b) Einleiten der wenigstens einen Flüssigkeit (2) über das Zufuhrelement (9) in Richtung der Austrittsöffnung (8), bis die wenigstens eine Flüssigkeit (2) mit dem immobilisierten Reaktionspartner in Kontakt steht.

17. Verfahren nach Anspruch 16,
weiter umfassend den Schritt
c) Einleiten einer zweiten Flüssigkeit und optional einer weiteren Flüssigkeit über das Zufuhrelement (9) in Richtung der Austrittsöffnung (8), bis die zweite Flüssigkeit mit dem immobilisierten Reaktionspartner in Kontakt steht.

18. Verfahren nach einem der Ansprüche 16 oder 17,
weiter umfassend den Schritt
d) Einleiten einer Waschlösung über das Zufuhrelement (9) in Richtung der Austrittsöffnung (8), bis die Waschlösung mit dem immobilisierten Reaktionspartner in Kontakt steht,
wobei Schritt d) vor und/oder nach jedem der Schritte a), b), oder c) durchgeführt werden kann.

19. Verfahren nach Anspruch 18, wobei das Einleiten der Waschlösung durchgeführt wird, bis die Vorrichtung nach einem der Ansprüche 1 bis 11 oder 14 bis 15 vollständig von Verunreinigungen befreit ist.

20. Verfahren nach einem der Ansprüche 16 bis 18,
wobei das Einleiten der wenigstens einen Flüssigkeit, der zweiten und optional weiteren Flüssigkeit und/oder Waschlösung, bevorzugt das Einleiten sowohl der wenigstens einen und zweiten und optional weiteren Flüssigkeit als auch der Waschlösung, gefolgt wird von einem zusätzlichen Schritt
e) Absaugen der wenigstens einen oder zweiten und optional weiteren Flüssigkeit oder der Waschlösung von dem immobilisierten Reaktionspartner aus Richtung der Adhäsionsfläche (14) über den Kanal (7).

## Claims

1. Device for contacting an immobilized reaction partner with at least one liquid, comprising
a trough (3) having a base (4) from which at least one elevation (5) having an upper side (6) projects into the interior of the trough (3),
wherein the at least one elevation (5) has a channel (7) which runs from the base (4) of the trough (3) to the upper side of the elevation (6), preferably perpendicularly to the base (4) of the trough (3), and opens into at least one escape opening (8) on the upper side of the elevation (6),
wherein the channel (7) is fed from at least one liquid reservoir (11) containing the at least one liquid (2) via a feed element (9) by means of a pumping device (10),
a holding device (12) configured to detachably hold at least one object carrier (13) having at least one adhesion surface (14) containing the immobilized reaction partner, such that the adhesion surface (14) is facing the interior of the trough (3), and that the upper side of the elevation (6) and the adhesion surface (14) are positioned relative to one another such that liquid (2) escaping from the escape opening (8) is in contact with the immobilized reagent,
wherein the device comprises at least one object carrier (13) inserted into the holding device (12),
wherein the upper side of the elevation (6) and the adhesion surface (14) are arranged in parallel to one another,
and wherein the object carrier (13) has at least two adhesion surfaces (14) separated from one another or the holding device (12) is provided as a separate part which is equipped to accommodate and to hold at least two object carriers (13),
**characterized in that** the device has two or more elevations (6) and accordingly two or more corresponding adhesion surfaces (14) in the one trough (3),
and **in that** these are shaped and situated at a sufficient distance from one another such that the liquid (2) introduced through the elevations (6) does not come into contact with adhesion surfaces (14) which are opposite adjacent elevations.

2. Device according to Claim 1,
wherein the channel (7) is additionally fed from a further liquid reservoir (11) containing a second liquid.

3. Device according to either of Claims 1 and 2,
wherein the channel (7) opens into more than one escape opening (8) on the upper side of the elevation (6).

4. Device according to any of Claims 1 to 3,
wherein the adhesion surface (14) has a central point (15) and the upper side of the elevation (6) has a central point (16),
and wherein the central point of the adhesion surface (15) and the central point of the upper side of the elevation (16) are opposite each other.

5. Device according to any of Claims 1 to 4,
wherein the trough (3) has at least one drain opening (17).

6. Device according to any of Claims 1 to 5,
wherein the holding device (12) allows a movement of the object carrier (13), preferably over at least one groove (18) or rail applied to the upper edges of the trough (3).

7. Device according to Claim 6, wherein the trough (3) is further provided with at least one air nozzle (20) for drying the adhesion surface (14).

8. Device according to any of Claims 1 to 7,
wherein the pumping device (10) allows any liquid (2) which is in contact with the immobilized reaction partner to be aspirated from the direction of the escape opening (8).

9. Device according to any of Claims 1 to 8,
further comprising a transport device which allows the insertion of the object carrier (13) into the device.

10. Device according to any of Claims 1 to 9, wherein the escape opening (8) on the upper side of the elevation (6), the upper side of the elevation (6) and the adhesion surface (14) are designed such that the at least one liquid (2) flows tangentially along the immobilized reaction partner when said liquid is introduced into the device.

11. Device according to any of Claims 1 to 10,
wherein the holding device (12) comprises a frame (21) equipped to accommodate and to hold at least one object carrier (13), preferably a multiplicity of object carriers (13), wherein, after accommodation, the adhesion surface (14) is situated on that side of the object carrier (13) which is resting on the frame (21).

12. Device according to any of Claims 1 to 11, wherein the relative position of trough (3) and object carriers (13) is determined by the holding device (12).

13. Device according to any of Claims 1 to 11, wherein the device is designed such that more liquid than can be held between adhesion surface (14) and upper side of the elevation (6) can run toward the sides of the elevation on the base (4) of the trough (3).

14. Device according to any of Claims 1 to 13, wherein the frame (21) has at least one lateral wall which is preferably provided with a handle (23).

15. Device according to Claim 14, wherein the lateral wall of the frame (21) has a profile suited to positioning the holding device (12) and thus the adhesion surface (14) in the device.

16. Method for contacting an immobilized reaction partner with at least one liquid, comprising the steps of
a) providing the device according to any of Claims 1 to 15 comprising at least one object carrier (13) containing immobilized reaction partner,
b) introducing the at least one liquid (2) in the direction of the escape opening (8) via the feed element (9) until the at least one liquid (2) is in contact with the immobilized reaction partner.

17. Method according to Claim 16,
further comprising the step of
c) introducing a second liquid and optionally a further liquid in the direction of the escape opening (8) via the feed element (9) until the second liquid is in contact with the immobilized reaction partner.

18. Method according to either of Claims 16 and 17,
further comprising the step of
d) introducing a wash solution in the direction of the escape opening (8) via the feed element (9) until the wash solution is in contact with the immobilized reaction partner,
wherein step d) can be carried out before and/or after each of steps a), b), or c).

19. Method according to Claim 18, wherein the introduction of the wash solution is carried out until the device according to any of Claims 1 to 11 or 14 to 15 has been completely cleared of contaminants.

20. Method according to any of Claims 16 to 18,
wherein the introduction of the at least one liquid, the second liquid and optionally further liquid and/or wash solution, preferably the introduction of both the at least one and second and optionally further liquid and the wash solution, is followed by an additional step of
e) aspirating the at least one or second and optionally further liquid or the wash solution from the immobilized reaction partner from the direction of the adhesion surface (14) via the channel (7).

## Revendications

1. Dispositif de mise en contact d'un réactif immobilisé avec au moins un liquide, le dispositif comprenant
une cuve (3) pourvue d'un fond (4) duquel au moins une élévation (5) fait saillie par un côté supérieur (6) dans l'espace intérieur de la cuve (3),
l'au moins une élévation (5) comportant un conduit (7) qui s'étend depuis le fond (4) de la cuve (3) vers le côté supérieur de l'élévation (6), de préférence perpendiculairement au fond (4) de la cuve (3), et qui débouche du côté supérieur de l'élévation (6) dans au moins une ouverture de sortie (8),
le conduit (7) étant alimenté à partir d'au moins un réservoir de liquide (11), qui contient l'au moins un liquide (2), au moyen d'un dispositif de pompage (10) par le biais d'un élément d'alimentation (9),
un dispositif de support (12) qui est conçu pour supporter de manière détachable au moins un porte-objet (13) comportant au moins une surface d'adhérence (14) avec le réactif immobilisé de telle sorte que la surface d'adhérence (14) soit dirigée vers l'espace intérieur de la cuve (3) et de telle sorte que le côté supérieur de l'élévation (6) et la surface d'adhérence (14) soient positionnés l'un par rapport à l'autre de manière à ce que le liquide (2) sortant de l'ouverture de sortie (8) soit en contact avec le réactif immobilisé,
le dispositif comprenant au moins une porte-objet (13) introduit dans le dispositif de support (12),
le côté supérieur de l'élévation (6) et la surface d'adhérence (14) étant disposés parallèlement l'un à l'autre,
et le porte-objet (13) comportant au moins deux surfaces d'adhérence (14) distinctes l'une de l'autre ou le dispositif de support (12) étant prévu en tant que partie séparée qui est destinée à recevoir et supporter au moins deux porte-objets (13),
**caractérisé en ce que** le dispositif comporte dans ladite cuve (3) deux élévations (6) ou plus et en conséquence deux surfaces d'adhérence correspondantes (14) ou plus,
et que celles-ci sont conçues, et sont situées à une distance suffisante les unes des autres, de telle manière que le liquide (2), introduit à travers les élévations (6), n'entre pas en contact avec des surfaces d'adhérence (14) qui sont situées en face d'élévations adjacentes.

2. Dispositif selon la revendication 1,
le conduit (7) étant en outre alimenté à partir d'un autre réservoir de liquide (11) qui contient un deuxième liquide.

3. Dispositif selon l'une des revendications 1 et 2,
le conduit (7) débouchant du côté supérieur de l'élévation (6) dans plus d'une ouverture de sortie (8).

4. Dispositif selon l'une des revendications 1 à 3,
la surface d'adhérence (14) comportant un centre (15) et le côté supérieur de l'élévation (6) comportant un centre (16),
et le centre de la surface d'adhérence (15) et le centre du côté supérieur de l'élévation (16) étant situé l'un en face de l'autre.

5. Dispositif selon l'une des revendications 1 à 4,
la cuve (3) comportant au moins une ouverture de vidange (17).

6. Dispositif selon l'une des revendications 1 à 5,
le dispositif de support (12) permettant un déplacement du porte-objet (13), de préférence par le biais d'au moins une rigole (18) ou d'un rail montés au niveau des bords supérieurs de la cuve (3).

7. Dispositif selon la revendication 6, la cuve (3) étant en outre équipée d'au moins une buse à air (20) destinée à sécher la surface d'adhérence (14).

8. Dispositif selon l'une des revendications 1 à 7,
le dispositif de pompage (10) permettant d'aspirer tout liquide (2), qui est en contact avec le réactif immobilisé, depuis la direction de l'ouverture de sortie (8).

9. Dispositif selon l'une des revendications 1 à 8,
le dispositif comprenant en outre un dispositif de transport qui permet d'introduire le porte-objet (13) dans le dispositif.

10. Dispositif selon l'une des revendications 1 à 9, l'ouverture de sortie (8) du côté supérieur de l'élévation (6), le côté supérieur de l'élévation (6) et la surface d'adhérence (14) étant conçus de telle sorte que l'au moins un liquide (2) s'écoule tangentiellement le long du réactif immobilisé lorsque ledit liquide est introduit dans le dispositif.

11. Dispositif selon l'une des revendications 1 à 10,
le dispositif de support (12) comprenant un cadre (21) qui est équipé pour recevoir et supporter au moins un porte-objet (13), de préférence une multitude de porte-objets (13), la surface d'adhérence (14) étant située, après la réception, du côté du porte-objet (13) avec lequel celui-ci est en appui sur le cadre (21).

12. Dispositif selon l'une des revendications 1 à 11, dans lequel la position relative de l'auge (3) et des glissières (13) est déterminée par le dispositif de maintien (12).

13. Dispositif selon l'une des revendications 1 à 11, le dispositif étant conçu de manière à ce que davantage de liquide que ce qui peut être retenu entre la surface d'adhérence (14) et le côté supérieur de l'élévation (6) puisse s'écouler sur le fond (4) de la cuve (3) en direction des côtés de l'élévation.

14. Dispositif selon l'une des revendications 1 à 13, le cadre (21) comportant au moins une paroi latérale qui est de préférence pourvue d'une poignée (23).

15. Dispositif selon la revendication 14, la paroi latérale du cadre (21) comportant un profil qui est adapté pour positionner le dispositif de support (12) et donc la surface d'adhérence (14) dans le dispositif.

16. Procédé de mise en contact d'un réactif immobilisé avec au moins un liquide, le procédé comprenant les étapes suivantes
a) fournir le dispositif selon l'une des revendications 1 à 15 comprenant au moins un porte-objet (13) pourvu de réactif immobilisé,
b) introduire l'au moins un liquide (2) par le biais de l'élément d'alimentation (9) en direction de l'ouverture de sortie (8) jusqu'à ce que l'au moins un liquide (2) soit en contact avec le réactif immobilisé.

17. Procédé selon la revendication 16, comprenant en outre l'étape suivante c) introduire un deuxième liquide et éventuellement un autre liquide par le biais de l'élément d'alimentation (9) en direction de l'ouverture de sortie (8) jusqu'à ce que le deuxième liquide soit en contact avec le réactif immobilisé.

18. Procédé selon l'une des revendications 16 et 17, comprenant en outre l'étape suivante
d) introduire une solution de lavage par le biais de l'élément d'alimentation (9) en direction de l'ouverture de sortie (8) jusqu'à ce que la solution de lavage soit en contact avec le réactif immobilisé,
l'étape d) pouvant être réalisée avant et/ou après chacune des étapes a), b) ou c).

19. Procédé selon la revendication 18, l'introduction de la solution de lavage étant effectuée jusqu'à ce que le dispositif selon l'une des revendications 1 à 11 ou 14 à 15 soit complètement débarrassé d'impuretés.

20. Procédé selon l'une des revendications 16 à 18,
l'introduction de l'au moins un liquide, du deuxième liquide et éventuellement de l'autre liquide et/ou de la solution de lavage, de préférence l'introduction aussi bien de l'au moins un liquide et du deuxième liquide et éventuellement de l'autre liquide que de la solution de lavage, étant suivie d'une étape supplémentaire suivante
e) aspirer l'au moins un liquide ou le deuxième liquide et éventuellement un autre liquide ou la solution de lavage du réactif immobilisé depuis la direction de la surface d'adhérence (14) par le biais du conduit (7).
